(19) Europäisches Patentamt
European Patent Office
Office européen des brevets

(11) **EP 3 454 063 B1**

(12) **EUROPÄISCHE PATENTSCHRIFT**

(45) Veröffentlichungstag und Bekanntmachung des Hinweises auf die Patenterteilung:
**18.05.2022 Patentblatt 2022/20**

(21) Anmeldenummer: **18192931.6**

(22) Anmeldetag: **06.09.2018**

(51) Internationale Patentklassifikation (IPC):
**G01N 33/50** (2006.01)  **G01N 33/543** (2006.01)
**G01N 33/68** (2006.01)

(52) Gemeinsame Patentklassifikation (CPC):
**G01N 33/54313; G01N 33/5091; G01N 33/5094; G01N 33/68**

(54) **DURCHFLUSSZYTOMETRIE-MESSVERFAHREN**

FLOW CYTOMETRY MEASURING METHOD

PROCÉDÉ DE MESURE DE CYTOMÉTRIE EN FLUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.09.2017 EP 17001496**

(43) Veröffentlichungstag der Anmeldung:
**13.03.2019 Patentblatt 2019/11**

(73) Patentinhaber: **AVA Lifescience GmbH**
**79211 Denzlingen (DE)**

(72) Erfinder:
• **KLAPPROTH, Holger**
**79108 Freiburg i. Br. (DE)**
• **BIRSNER, Ulrich**
**79110 Freiburg (DE)**
• **KESSEMEIER, Marc**
**79312 Emmendingen (DE)**

(74) Vertreter: **Huwer, Andreas**
**Huwer & Partner**
**Patent- und Rechtsanwälte PartG mbB**
**Schwaighofstrasse 1**
**79100 Freiburg (DE)**

(56) Entgegenhaltungen:
WO-A1-2012/117091  WO-A1-2013/064685
WO-A2-2007/134189  US-A1- 2010 015 643

• **WEIGUM S E ET AL: "Cell-based sensor for analysis of EGFR biomarker expression in oral cancer", LAB ON A CHIP, ROYAL SOCIETY OF CHEMISTRY, Bd. 7, Nr. 8, 1. August 2007 (2007-08-01), Seiten 995-1003, XP002550801, ISSN: 1473-0197, DOI: 10.1039/B703918B [gefunden am 2007-07-11]**
• **MAXIME MOULARD ET AL: "How validated receptor occupancy flow cytometry assays can impact decisions and support drug development : HOW VALIDATED RO FLOW CYTOMETRY ASSAYS CAN IMPACT DECISIONS AND SUPPORT DRUG DEVELOPMENT", CYTOMETRY. PART B, CLINICAL CYTOMETRY, Bd. 90, Nr. 2, 11. Dezember 2015 (2015-12-11), Seiten 150-158, XP055383541, US ISSN: 1552-4949, DOI: 10.1002/cyto.b.21320**
• **XIAOSAI YAO ET AL: "Determination of 35 cell surface antigen levels in malignant pleural effusions identifies CD24 as a marker of disseminated tumor cells : CD24 as A Marker of Disseminated Tumor Cells", INTERNATIONAL JOURNAL OF CANCER, 1. Juni 2013 (2013-06-01), Seiten 2925-2933, XP055521745, US ISSN: 0020-7136, DOI: 10.1002/ijc.28312**
• **OBA R ET AL: "Quantitative analysis of Toll-like receptor 2 on circulating monocytes in patients with infectious diseases by using flow cytometry", NIHON MEN'EKI GAKKAI SOKAI, GAKUJUTSU SHUKAI KIROKU // PROCEEDINGS OF THE JAPANESE SOCIETY FOR IMMUNOLOGY (JSI), NIHON MEN'EKI GAKKAI // JAPANESE SOCIETY FOR IMMUNOLOGY (JSI), JP, Bd. 36, 15. November 2006 (2006-11-15), XP003019429, ISSN: 0919-1984**

**Beschreibung**

Die Erfindung betrifft ein Durchflusszytometrie-Messverfahren, bei dem ein Analysemedium bereitgestellt wird, welches ein Fluid und darin enthaltene, zu klassifizierende biologische Zellen aufweist, wobei mindestens ein Markierungsmolekül bereitgestellt und derart mit dem Analysemedium in Kontakt gebracht wird, dass das Markierungsmolekül spezifisch an mindestens eine an der Oberfläche der Zellen befindliche Zielstruktur binden kann, wenn die Zelle diese Zielstruktur aufweist, wobei ein Fluidstrom des Analysemediums erzeugt wird, in dem die Zellen einzeln in einen Messbereich eines Energiestrahls und/oder eines elektrischen Felds gelangen, wobei für die einzelnen, in dem Messbereich befindlichen Zellen jeweils zumindest für einen ersten physikalischen Parameter ein erster und für einen zweiten physikalischen Parameter ein zweiter Durchflusszytometrie-Messwert erfasst werden, wobei zumindest der erste Parameter eine Fluoreszenzstrahlung ist, welche das mindestens eine Markierungsmolekül bei Anregung mit dem Energiestrahl oder dem elektrischen Feld abgibt, und wobei die Zellen anhand der Durchflusszytometrie-Messwerte klassifiziert werden.

Der Begriff Durchflusszytometrie (Zytometrie: Zell-Vermessung) beschreibt ein Messverfahren, das in der Biologie und in der Medizin zur Anwendung kommt. Es erlaubt die Analyse von biologischen Zellen, die mit hoher Geschwindigkeit einzeln ein elektrisches Feld oder einen Lichtstrahl passieren. Je nach Form, Struktur und/oder Färbung der Zellen werden unterschiedliche Durchflusszytometrie-Messwerte erfasst, aus denen die Eigenschaften der einzelnen Zellen abgeleitet und die Zellen somit klassifiziert werden können.

Bei einer Form der Durchflusszytometrie werden Fluoreszenz-markierte Zellen je nach Färbung in unterschiedliche Reagenzgefäße sortiert. Entsprechende Geräte werden als Fluss-Sortierer (flow sorter) oder als FACS (fluorescence-activated cell sorting) bezeichnet. Manchmal wird der Begriff FACS (=fluorescence-activated cell scanning) mit dieser Bedeutung für Geräte verwendet, die keine Sortierung der Zellen, sondern nur eine Analyse oder Klassifizierung ihrer Eigenschaften durchführen.

Das Prinzip der Untersuchung beruht auf der Emission von optischen Signalen seitens der Zelle, wenn diese einen Laserstrahl passiert. Durch einen Hüllstrom fokussiert, tritt die Probe in den Mikrokanal einer hochpräzisen Küvette aus Glas oder Quarz ein, derart, dass jede Zelle einzeln nacheinander durch den Messbereich eines Laserstrahls geführt wird. Das dabei entstehende Streulicht oder Fluoreszenzsignal wird von einem Detektor erfasst. Das Ergebnis sind quantitative Informationen über jede einzelne Zelle. Durch die Analyse einer großen Anzahl von Zellen innerhalb eines sehr kurzen Zeitintervalls (>1000 Zellen/Sekunde) erhält man schnell repräsentative Informationen über Zell-Populationen, die in dem Analysemedium enthalten sind.

Zugleich mit dem gestreuten Licht kann man im Durchflusszytometer Fluoreszenzfarben messen. Nur wenige Zellen emittieren per se fluoreszierendes Licht. Daher verwendet man Markierungsmoleküle, wie z.B. Farbstoffe, die an bestimmte Zielstrukturen der Zellen binden. Setzt man z.B. die Farbstoffe DAPI (4',6-Diamidine-2'-phenylindole dihydrochloride) und Propidiumiodid ein, welche an die Desoxyribonukleinsäure (DNA) einer Zelle binden (DAPI) bzw. in diese interkalieren (Propidiumiodid) - d. h. sich zwischen die Basen einlagern -, kann man anhand der Helligkeit der Fluoreszenzstrahlung, die das mindestens eine, an die Zelle immobilisierte Markierungsmolekül beim Passieren des Laserstrahls aussendet, untersuchen, wie viel DNA die Zelle enthält.

Auch Antikörper, die mit Fluoreszenzfarbstoffen markiert sind, können als Markierungsmoleküle verwendet werden. Die Antikörper sind meist gegen bestimmte Oberflächenproteine gerichtet, z.B. Proteine der CD-Klassifizierung (CD: Cluster of differentiation). Nach Markierung kann bei Bedarf dann auch die Sortierung der Zellen nach diesen Merkmalen erfolgen. Durch Einsatz von verschiedenfarbigen Lasern und vor allem Filtern kann die Anzahl der einsetzbaren Farbstoffe und damit die Informationsdichte erhöht werden. Dabei sind Antikörper die am häufigsten verwendeten Moleküle, um Oberflächenproteine auf Zellen zu markieren. Unter Markierungsmolekülen werden Antikörper, die mit mindestens einem optischen Markern markiert sind, und/oder andere Moleküle verstanden, die geeignet sind, spezifisch an eine an der Oberfläche einer in dem Analysemedium enthaltenen Zelle, wie zum Beispiel einer Krebszelle, befindliche Zielstruktur zu binden und die Zelle optisch zu markieren.

Obwohl das Durchflusszytometer bei fluoreszenzmarkierten Zellen sowohl die Messung der Anzahl der markierten Zellen als auch die Erfassung eines Messwerts für das von den einzelnen Zellen abgegebene Fluoreszenzsignal ermöglicht, ist eine quantitative Auswertung des Fluoreszenzsignals schwierig, da die für die Messungen verwendeten Durchflusszytometer unterschiedlich sind und daher bei Messungen, die mit verschiedenen Durchflusszytometern vorgenommen wurden, eine Vergleichbarkeit der Durchflusszytometrie-Messwerte nicht gewährleistet ist. Insbesondere werden die Durchflusszytometrie-Messwerte durch die Intensität der zur Beleuchtung des Messbereichs verwendeten Laserstrahlung, durch Toleranzen von optischen Filtern, dem Aufbau der Durchflussmesszelle (Fluidik), den verwendete Reagenzien, Detektoren und der Variabilität der die Markierungsmoleküle enthaltenden Proben beeinflusst.

Zwar ist eine Quantifizierung der Fluoreszenzmesswerte der Zellen durch Vergleich des ersten Durchflusszytometrie-Messwerts mit einem Schwellwert möglich, jedoch wird dieser meistens willkürlich gesetzt. Eine genaue Quantifizierung der Bindungsereignisse pro Zelle und damit eine universelle plattformunabhängige Vergleichbarkeit der Fluoreszenzmesswerte ist bisher nicht möglich.

Es ist auch bereits bekannt, Durchflusszytometrie-Messgeräte mit Hilfe von fluoreszierenden Mikropartikeln, die in einem Fluid angeordnet sind, das durch den Messbereich des Energiestrahls bzw. des elektrischen Felds geleitet wird, zu kalibrieren. Es zeigte sich jedoch, dass diese Kalibrierung nicht ausreicht, um einen genauen Vergleich der mit verschiedenen Durchflusszytometern gemessenen Fluoreszenzmesswerte zu ermöglichen.

Die US 2010/015643 A1 offenbart ein Durchflusszytometrie-Messverfahren zur Quantifizierung von Zielstrukturen auf Zellen, wobei eine Kalibrierung mittels mindestens zwei verschiedener Partikel (beads) erfolgt. Diese Partikel weisen auf ihrer Oberfläche eine bekannte, aber per Partikel variierende Menge der Zielstruktur auf. Die Partikel werden mit dem Markierungsmolekül in Kontakt gebracht und in einem Fluidstrom einzeln nacheinander in den Messbereich eines Fluoreszenz-Durchflusszytometers eingebracht. Es wird eine Kalibrierungskurve erstellt und die Fluoreszenzintensität der analysierten Zellen wird anhand der Kalibrierungskurve umgerechnet in die Anzahl der Zielstrukturen auf der jeweiligen Testzelle, wobei die Anzahl der Erkennungsstellen pro Zelle numerisch ausgedrückt und normalisiert werden kann. Als Negativkontrolle wird ein Partikel verwendet der mit einem nichtmarkierten Markierungsmolekül kontaktiert wurde, so dass die Zielstrukturen auf dem Partikel blockiert sind und nicht mehr mit dem eigentlichen Markierungsmolekül reagieren können.

Aufgabe der Erfindung ist es daher, ein Durchflusszytometrie-Messverfahren der eingangs genannten Art zur Verfügung zu stellen, das es ermöglicht, die Zielstruktur an der Oberfläche der Zellen mit großer Präzision quantitativ nachzuweisen. Außerdem sollen die durch Fluoreszenzmessung erhaltenen quantitativen Messwerte, wenn sie mit verschiedenen Durchflusszytometern gemessen wurden, präzise miteinander vergleichbar sein.

Diese Aufgabe wird bezüglich des Durchflusszytometrie-Messverfahrens mit den Merkmalen des Anspruchs 1 gelöst. Der Erfindung liegt die Erkenntnis zu Grunde, dass die Toleranzen der für den Nachweis der Bindung der Markierungsmoleküle an die Zielstruktur der Zellen verwendeten Reagenzien, die Variabilität der Markierungsmoleküle und die Zusammensetzung des Analysemediums einen wesentlichen Einfluss auf die Durchflusszytometrie-Messwerte der zu klassifizierenden Zellen hat. Diese Toleranzen werden insbesondere durch das Alter der Markierungsmoleküle, die Art und Weise, wie und unter welchen Bedingungen die Markierungsmoleküle vor der Durchführung des Durchflusszytometrie-Messverfahrens gelagert wurden, und durch zusätzlich zu den Zellen in dem Analysemedium enthaltene Substanzen, wie z.B. Inhibitoren, welche die Bindung der Markierungsmoleküle an die Zielstruktur erschweren oder anstelle der Markierungsmoleküle an die Zielstruktur binden, beeinflusst. Außerdem können auch Umgebungsbedingungen, wie z.B. die Temperatur, die Durchflusszytometrie-Messwerte der Zellen beeinflussen.

Diese Einflüsse werden bei dem erfindungsgemäßen Verfahren dadurch kompensiert, dass standardisierte erste und zweite Kalibratoren bereitgestellt werden, dass für diese Kalibratoren entsprechende Durchflusszytometrie-Messwerte erfasst werden wie für die Zellen, und dass die an den Zellen gemessenen Durchflusszytometrie-Messwerte mit Hilfe der Durchflusszytometrie-Messwerte der Kalibratoren normalisiert werden. Hierdurch wird eine genaue Quantifizierung der zwischen den einzelnen Zellen und den Markierungsmolekülen bzw. deren Antikörpern auftretenden Bindungsereignisse pro Zelle ermöglicht. Die normalisierten Durchflusszytometrie-Messwerte können auch bei Durchflusszytometrie-Messungen, die mit unterschiedlichen Durchfluss-Zytometern durchgeführt wurden und/oder bei denen Reagenzien bzw. Markierungsmolekülen verwendet wurden, die bezüglich ihrer Eigenschaften voneinander abweichen, exakten miteinander verglichen werden. Hierdurch ist es insbesondere möglich, durch Clusteranalyse zusammengehörige Zell-Subpolulationen zu identifizieren. Da die Kalibratoren keine lebenden Zellen sind sondern Festkörperpartikel als Träger für die mindestens eine Zielstruktur aufweisen, sind die Kalibratoren langzeitstabil, d.h. sie lassen sich über einen längeren Zeitraum lagern, ohne dass sich ihre für den Nachweis der Zielstruktur relevanten Eigenschaften wesentlich verändern.

Bei der Erfindung werden mindestens zwei Kalibratorarten von ersten Kalibratoren bereitgestellt und mit dem Analysemedium vermischt, wobei sich die Kalibratoren der unterschiedlichen Kalibratorarten insbesondere hinsichtlich der Flächenbelegungsdichte und/oder der Anordnung ihrer mindestens einen Zielstruktur auf der Oberfläche des Festkörperpartikels derart voneinander unterscheiden, dass die ersten Durchflusszytometrie-Messwerte einer ersten Kalibratorart eine Signalstärke aufweisen, die größer ist als die Signalstärke der ersten Durchflusszytometrie-Messwerte der zweiten Kalibratoren und kleiner ist als die Signalstärke der ersten Durchflusszytometrie-Messwerte einer zweiten Kalibratorart. Hierdurch kann der Einfluss eines nichtlinearen Signalverlaufs bei der Erfassung der Durchflusszytometrie-Messwerte kompensiert werden.

Der Parameterraum jeder Analyse ist von der Anzahl der maximalen Bindungsereignisse pro Zelle und den Messparametern abhängig. D.h. bei der Bestimmung von z.B. drei Parametern ergeben diese einen dreidimensionalen Raum, dessen Achsen aber nicht zwingend rechtwinkelig zueinander stehen. Die Markierungsart und Markierungsdichte (Anzahl der Fluorophore pro Antikörper) und die Bindestärke und Spezifität der verwendeten Antikörper (bzw. Markierungsmoleküle) spielen dabei ebenfalls eine Rolle.

Bei dem erfindungsgemäßen Verfahren werden normalisierte Durchflusszytometrie-Messwerte für die Zellen bereitgestellt, die unabhängig von den physikalischen Eigenschaften des für die Durchführung des Verfahrens verwendeten Durchflusszytometers, unabhängig von Toleranzen von den verwendeten Reagenzien und Markierungsmoleküle und unabhängig von Substanzen und Wirkstoffen ist, die in dem Analysemedium zusätzlich zu den Zellen enthalten sein können.

Zur Bildung des normalisierten ersten Durchflusszytometrie-Messwerts wird die Differenz zwischen dem ersten Durchflusszytometrie-Messwert des ersten Kalibrators und dem ersten Durchflusszytometrie-Messwert des zweiten Kalibrators zur Differenz zwischen dem ersten Durchflusszytometrie-Messwert der Zelle und dem ersten Durchflusszytometrie-Messwert des zweiten Kalibrators ins Verhältnis gesetzt. Dabei beträgt der normalisierte Durchflusszytometrie-Messwert der Zellen vorzugsweise 100%, wenn der erste Durchflusszytometrie-Messwert der Zellen mit dem ersten Durchflusszytometrie-Messwert des ersten Kalibrators übereinstimmt. Wenn der erste Durchflusszytometrie-Messwert der Zellen mit dem ersten Durchflusszytometrie-Messwert des zweiten Kalibrators übereinstimmt, hat der normalisierte Durchflusszytometrie-Messwert der Zellen den Wert null. Die ersten Kalibratoren sind bevorzugt derart ausgestaltet, dass der erste Durchflusszytometrie-Messwert mindestens eines ersten Kalibrators mit dem zu erwartenden Maximalwert des ersten Durchflusszytometrie-Messwerts der Zellen übereinstimmt oder etwas darüberliegt, insbesondere maximal 3%, maximal 5%, maximal 10% oder maximal 25% über dem erwarteten ersten Durchflusszytometrie-Messwert des ersten Kalibrators. Der entsprechende Erwartungswert kann experimentell ermittelt werden.

Die vorstehend genannte Aufgabe wird auch mit den Merkmalen des Patentanspruchs 2 gelöst. Dabei werden mehrere identische erste Kalibratoren und mehrere identische zweite Kalibratoren bereitgestellt und mit dem Analysemedium vermischt, bevor die Durchflusszytometrie-Messwerte für die einzelnen erfasst werden, wobei aus den ersten Durchflusszytometrie-Messwerten der ersten Kalibratoren ein gemittelter erster Durchflusszytometrie-Messwert für die ersten Kalibratoren und aus den ersten Durchflusszytometrie-Messwerten der zweiten Kalibratoren ein gemittelter erster Durchflusszytometrie-Messwert für die zweiten Kalibratoren gebildet wird, und wobei zur Bildung eines normalisierten ersten Messwerts einer Zelle die Differenz zwischen dem gemittelten ersten Durchflusszytometrie-Messwert der ersten Kalibratoren und dem gemittelten ersten Durchflusszytometrie-Messwert der zweiten Kalibratoren zur Differenz zwischen dem ersten Durchflusszytometrie-Messwert der Zelle und dem gemittelten ersten Durchflusszytometrie-Messwert der zweiten Kalibratoren ins Verhältnis gesetzt wird

Zur Bildung eines normalisierten ersten Messwerts für eine mehrere Zellen umfassende Zellpopulation kann jeweils für die einzelnen Zellen erste Durchflusszytometrie-Messwerte erfasst werden, wobei aus diesen Durchflusszytometrie-Messwerten ein gemittelter erster Durchflusszytometrie-Messwert für die Zellpopulation gebildet wird, und wobei zur Bildung des normalisierten ersten Messwerts für eine Mehrzahl von Zellen die Differenz zwischen dem gemittelten ersten Durchflusszytometrie-Messwert der ersten Kalibratoren und dem gemittelten ersten Durchflusszytometrie-Messwert der zweiten Kalibratoren zur Differenz zwischen dem ersten gemittelten Durchflusszytometrie-Messwert der Zellpopulation und dem gemittelten ersten Durchflusszytometrie-Messwert der zweiten Kalibratoren ins Verhältnis gesetzt wird. Dabei kann der Mittelwert mit an sich bekannten statistischen Verfahren bestimmt werden und insbesondere der arithmetische Mittelwert sein. Bei Bedarf kann der normalisierte erste Messwert zusätzlich skaliert werden, wobei der Skalierungsfaktor beispielsweise dem Quotient aus dem gemittelten ersten Durchflusszytometrie-Messwert der ersten Kalibratoren und der Zahl 100 entsprechen kann.

Bei einer Weiterbildung der Erfindung wird für mindestens einen ersten Kalibrator ein erster Kalibrierfaktor bereitgestellt, der bezüglich des Messignals für den ersten Parameter dem Verhältnis zwischen der Messsignalstärke des ersten Kalibrators und der Messsignalstärke eines die Zielstruktur aufweisenden ersten Referenz-Kalibrators entspricht, wobei für den ersten physikalischen Parameter des ersten Kalibrators ein erstes Messsignal erfasst wird und der erste Durchflusszytometrie-Messwert des ersten Kalibrators aus dem ersten Messsignal und dem ersten Kalibrierfaktor gebildet wird, wobei für den zweiten Kalibrator ein zweiter Kalibrierfaktor bereitgestellt wird, der bezüglich des Messignals für den ersten Parameter dem Verhältnis zwischen der Messsignalstärke des zweiten Kalibrators und der Messsignalstärke eines die Zielstruktur nicht aufweisenden zweiten Referenz-Kalibrators entspricht, und wobei für den ersten physikalischen Parameter des zweiten Kalibrators ein zweites Messsignal erfasst wird und der erste Durchflusszytometrie-Messwert des zweiten Kalibrators aus dem zweiten Messsignal und dem zweiten Kalibrierfaktor gebildet wird.

Durch die Kalibrierfaktoren ist es möglich, Durchflusszytometrie-Messwerte, die mit unterschiedlichen, voneinander abweichenden Chargen des ersten Kalibrators und/oder des zweiten Kalibrators durchgeführt wurden, noch präziser miteinander zu vergleichen. Die Kalibrierfaktoren werden bevorzugt experimentell unter exakt definierten Bedingungen gemessen.

Hierbei wird zunächst für die ersten und zweiten Referenz-Kalibratoren, die in einer ersten Charge hergestellt werden, jeweils ein Erwartungswert für den ersten Durchflusszytometrie-Messwert bestimmt. Dabei wird anstelle des Analysemediums ein Puffer mit definierten, konstanten Eigenschaften verwendet, in dem die ersten bzw. zweiten Referenz-Kalibartoren angeordnet sind. Biologische Zellen sind in dem Puffer nicht enthalten. In diesem Puffer werden für eine Vielzahl von gleichen oder im Wesentlichen gleichen ersten bzw. zweiten Referenz-Kalibratoren jeweils die entsprechenden ersten Durchflusszytometrie-Messwerte gemessen. Aus den so erhaltenen Referenz-Durchflusszytometrie-Messwerten wird mit an sich bekannten Methoden der Statistik für die ersten und zweiten Referenz-Kalibrator jeweils ein Erwartungswert bestimmt.

In einem weiteren Schritt werden für erste und zweite Kalibratoren einer zweiten Charge in entsprechender Weise Erwartungswerte bestimmt.

Danach wird der erste Kalibrierfaktor ermittelt, indem der Quotient aus dem Erwartungswert der ersten Durchflusszytometrie-Messwerte der ersten Kalibratoren der zweiten Charge und dem Erwartungswert der ersten Durchflusszytometrie-Messwerte der ersten Referenz-Kalibratoren gebildet wird. In entsprechender Weise wird der zweite Kalibrierfaktor ermittelt, indem der Quotient aus dem Erwartungswert der ersten Durchflusszytometrie-Messwerte der zweiten Kalibratoren der zweiten Charge und dem Erwartungswert der ersten Durchflusszytometrie-Messwerte der zweiten Referenz-Kalibratoren gebildet wird.

Vorteilhaft ist, wenn in dem Fluid mindestens zwei unterschiedliche Populationen von Zellen enthalten sind, die sich derart voneinander unterscheiden, dass die ersten Durchflusszytometrie-Messwerte der Zellen einer ersten Population in einem ersten Signalstärkebereich und die die ersten Durchflusszytometrie-Messwerte der Zellen einer zweiten Population in einem außerhalb des ersten Signalstärkebereichs befindlichen zweiten Signalstärkebereich liegen, und wenn die Signalstärke der ersten Durchflusszytometrie-Messwerte der ersten Kalibratorart derart gewählt wird, dass sie zwischen dem ersten und zweiten Signalstärkebereich liegt. Der Kalibrator der ersten Kalibratorart erzeugt dabei eine Signalstärke, die es erlaubt, die Referenzbereiche von zwei Subpopulationen voneinander abzugrenzen. D.h. Zellen mit einem ersten Durchflusszytometrie-Messwert, der kleiner ist als der erste Durchflusszytometrie-Messwert des ersten Kalibrators der ersten Kalibratorart, gehören zu einer ersten Population und Zellen mit einem höheren Durchflusszytometrie-Messwert zu einer zweiten Population.

Bei einer bevorzugten Ausgestaltung der Erfindung ist die größte Abmessung der Festkörperpartikel kleiner als 20 $\mu$m, insbesondere kleiner als 15 $\mu$m und bevorzugt kleiner als 10 $\mu$m ist und/oder die kleinste Abmessung der Festkörperpartikel ist größer als 4 $\mu$m, insbesondere größer als 5 $\mu$m und bevorzugt größer als 6 $\mu$m. Geeignete Festkörperpartikel sind beispielsweise Mikrosphären mit einem Durchmesser von 6-10 $\mu$m. Die Festkörperpartikel haben bevorzugt eine ähnliche Größe wie die in dem Analysemedium befindlichen Zellen.

Die Festkörperpartikel sind bevorzugt synthetische Mikropartikel. Die Festkörperpartikel können ein Polystyren, Melamin, Latex und/oder Silikat aufweisen oder aus einem dieser Materialien bestehen.

Die Markierungsmoleküle können Moleküle sein, die an Zielantigene auf der Oberfläche von Zellen oder Kalibratoren binden und anschließend zum Nachweis dieser Zielantigene verwendet werden können. Solche Moleküle sind z.B. Antikörper und deren Fragmente, Lektine, andere bindende Proteine (z.B.Protein A).

Die Zielantigene auf den ersten Kalibratoren sind bevorzugt kovalent immobilisiert. Verfahren zum Herstellen einer solchen kovalenten Bindung sind aus dem Stand der Technik bekannt und umfassen z.B. Bindung an Amine (über Crosslinker), Thiole, Epoxide, Aldehyde, Maleinimide und andere Gruppen. Geeignete Verfahren und deren chemische Umsetzung sind in der Fachliteratur beschrieben (Bioconjugate Techniques, Third Edition 3rd edition by Hermanson, Greg T. (2013)). Es können aber auch nicht kovalente Verfahren angewandt werden, wie z.B. His-Tag, Biotin-Streptavidin, Protein G, Protein A, vorimmobilisierte Antikörper.

Zielantigene bzw. Zielstrukturen auf den ersten Kalibratoren können beispielsweise Proteine, Peptide, Rezeptoren, Allergene, glykosylierte Proteine, Liposaccharide, Oligo- und Polysacharide, Nukleinsäuren sowie die Fragmente und Derivate der vorgenannten Substanzen sein.

Bei einer bevorzugten Ausführungsform der Erfindung umfasst der mindestens eine zweite Durchflusszytometrie-Messwert einen Vorwärtsstreuungs-Messwert für eine in dem Messbereich auftretende Vorwärtsstreuung des Energiestrahls und/oder einen Seitwärtsstreuungs-Messwert für eine in dem Messbereich auftretende Seitwärtsstreuung des Energiestrahls. Diese Messwerte lassen sich auf einfache Weise mittels geeigneter Sensoren erfassen. Der Vorwärtsstreuungs-Messwert ist ein Maß für die Größe und der Seitwärtsstreuungs-Messwert ein Maß für die Granularität der Zellen bzw. der Kalibratoren. Anhand dieser Messgrößen lassen sich die Zellen von den Kalibratoren unterscheiden. Somit lassen sich die gemessenen Durchflusszytometrie-Messwerte anhand der Vorwärts- und Seitwärtsstreuungs-Messwerte eindeutig den Zellen und den Kalibratoren zuordnen.

Es ist aber auch möglich, dass der mindestens eine zweite Durchflusszytometrie-Messwert ein Fluoreszenzmesswert für eine von den Markierungsmolekülen ausgesandte Fluoreszenzstrahlung ist, welche sich von der Fluoreszenzstrahlung des ersten Durchflusszytometrie-Messwerts unterscheidet. Dabei sind bevorzugt die Wellenlängen der Fluoreszenzstrahlung der ersten und zweiten Durchflusszytometrie-Messwerte unterschiedlich.

Vorteilhaft ist, wenn sowohl für die Zellen als auch für die ersten und zweiten Kalibratoren jeweils für mehrere Messkanäle mindestens ein erster Durchflusszytometrie-Messwert erfasst wird, wenn zumindest eine der Anzahl der Messkanäle entsprechende Anzahl von unterschiedlichen Markierungsmolekülen bereitgestellt und mit dem Analysemedium in Kontakt gebracht wird, und wenn der mindestens eine erste Kalibrator zumindest eine der Anzahl der Messkanäle entsprechende Anzahl von Zielstrukturen aufweist, die jeweils spezifisch an eines der unterschiedlichen Markierungsmoleküle binden, wenn sie mit dem betreffenden Markierungsmolekül in Kontakt gelangen.

Es ist aber auch denkbar, dass sowohl für die Zellen als auch für die ersten und zweiten Kalibratoren jeweils für mehrere Messkanäle mindestens ein erster Durchflusszytometrie-Messwert erfasst wird, dass zumindest eine der Anzahl der Messkanäle entsprechende Anzahl von unterschiedlichen Markierungsmolekülen bereitgestellt und mit dem Analysemedium in Kontakt gebracht wird, dass zumindest eine der Anzahl der Messkanäle entsprechende Anzahl von unterschiedlichen ersten Kalibratoren bereitgestellt und mit dem Analysemedium vermischt wird, und diese ersten Kalibratoren unterschiedliche Zielstrukturen aufweisen, die jeweils für eines der unterschiedlichen Markierungsmoleküle bindungsspezifisch sind.

Die Verwendung mehrerer Messparameter und deren Kalibratoren ist dabei nur durch die Anzahl der Messkanäle des Messgerätes und die verfügbaren Fluorophore beschränkt. Routinemäßig können sechs bis acht Parameter erfasst werden. Für die Analyse von AML-Zellen werden heute schon 30 relevante Parameter erfasst, die aber in mehreren Messdurchgängen erfasst werden müssen. Mit modernen Geräten, die auch die Messung im nahen Infrarotbereich erlauben, lassen sich deutlich mehr als acht Parameter erfassen. Sollen in einem solchen Parameterraum Subklassen von Zellen erfasst werden, so ist die Auflösung der Messung wichtig.

Dadurch, dass die ersten Kalibratoren (relative Signalstärke 100%) es erlauben, das zu erwartende Maximalsignal gerätespezifisch zu erfassen, kann die Auflösung erhöht werden (z.B. durch Anpassung der Laserleistung bzw. der Verstärkerleistung des Detektors, so dass messtechnisch das Maximale an Auflösung aus der Messung geholt werden kann). Mit einem derart erfassten und aufgearbeiteten Signal lassen sich nun Zellpopulationen mit Hilfe mathematischer Verfahren erfassen (z.B. Clusteranalyse - ein Standardverfahren der Mathematik, das unter https://de.wikipedia.org/wiki/Clusteranalyse beschrieben ist). Somit können sehr variable Erkrankungen wie z.B. Leukämien besser typisiert werden und damit auch besser therapiert werden, da entsprechende Marker für das Ansprechen von Therapien erfasst und bewertet werden können. Im besonderen Falle von Lymphomen und Leukämien sind eine Reihe von Markern bekannt, welche einen starken Einfluss auf die Prognostik der Erkrankung haben. So belegen aktuelle Studien, dass neben bekannten Markern wie CD38, die Expression von CD49d eine sehr schwerwiegende Auswirkungen auf den Verlauf der chronisch lymphatischen Leukemie (CLL) hat (DOI: 10.1111/j.1365-2141.2011.08725.x).

Bei einer bevorzugten Ausgestaltung der Erfindung weist die Zielstruktur auf den ersten Kalibratoren mindestens ein Antigen auf, das vorzugsweise ein mindestens ein Protein und/oder mindestens ein Peptid und/oder mindestens einen Rezeptor und/oder mindestens ein Allergen und/oder mindestens ein glykosyliertes Protein und/oder mindestens ein Liposaccharid und/oder mindestens ein Oligosacharide und/oder mindestens ein Polysaccharid und/oder mindestens eine Nukleinsäure und/oder mindestens ein Fragment und/oder Derivat der vorgenannten Substanzen umfasst.

Bei einer vorteilhaften Ausführungsform der Erfindung ist die Zielstruktur des mindestens einen ersten Kalibrators über mindestens eine aktivierte Carboxy-Gruppen und/oder mindestens eine aktivierte NH2-Gruppe am Festkörperpartikel des ersten Kalibrators immobilisiert. Dies ermöglicht eine stabile Kopplung und Immobilisierung der Zielstruktur, wie z.B. eines Antigens, an den Festköperpartikel. Entsprechende Mikropartikel sind kommerziell erhältlich.

Vorteilhaft ist, wenn mindestens eine auf dem Festkörperpartikel des ersten Kalibrators immobilisierte Zielstruktur mit einer der Zielstruktur übereinstimmt, die für Zellen mindestens einer Blutkrebsart typisch ist. Das erfindungsgemäße Verfahren kann dann zur Klassifizierung von Leukemie-Zellen verwendet werden. Insbesondere können dabei die schnellwachsenden Typ M Zellen von den weniger aggressiven Typ D Zellen unterschieden werden.

Bei einer bevorzugten Ausgestaltung der Erfindung stimmt die mindestens eine auf dem Festkörperpartikel des ersten Kalibrators immobilisierte Zielstruktur mit einer der Zielstruktur übereinstimmt, die innerhalb des B Zell Rezeptors (BCR) auf der Oberfläche von chronisch lymphatischen Leukemie-Zellen (CLL-Zellen) vorkommt. Diese Leukemie-Zellen sind besonders aggressiv. Durch ihre hohe Affinität und Spezifität ist diese Zielstruktur gut für eine durchflusszytometrische Diagnostik (FACS) geeignet.

Die Kalibratoren werden bei der Messung dem Analysemedium, z.B. Blut, zugegeben. Geeignete Konzentrationen der Kalibratoren sind bevorzugt $1\times10^5$ pro $1\times10^6$ Zellen. Bei der Messung werden die Kalibratoren über die Parameter Forward-Scatter (Größe) und Sideward-Scatter (Granularität) identifiziert und somit von den Zielzellen getrennt.

Weitere Einzelheiten, Merkmale und Vorteile der vorliegenden Erfindung ergeben sich aus der nachfolgenden Beschreibung von Ausführungsbeispielen unter Bezugnahme auf die Zeichnung. Es zeigt:

Fig. 1    eine graphische Darstellung von mit Hilfe eines Durchflusszytometers für eine Population von zweiten Kalibratoren (Nullkalibrator) erfassten Messwerten, wobei auf der Abszisse die gemessene Intensität PE und auf der Abszisse die Anzahl n der Bindungsereignisse aufgetragen ist,

Fig. 2    eine Darstellung ähnlich Fig. 1 wobei jedoch die Messwerte für eine Population von ersten Kalibratoren einer ersten Kalibratorart dargestellt sind,

Fig. 3    eine Darstellung ähnlich Fig. 1 wobei jedoch die Messwerte für eine Population von ersten Kalibratoren einer zweiten Kalibratorart dargestellt sind, und

Fig. 4    eine Standardkurve, bei der auf der Abszisse die Menge Protein CD23 pro $2{,}1\cdot10^6$ Festkörperpartikel und auf der Ordinate der MFI-Wert (mittlerer Fluoreszenz-Messwert) aufgetragen ist.

Bei einem ersten Ausführungsbeispiel der Erfindung werden als Zielstrukturen krankheitsassoziierte Antigene bereitgestellt. Als eine erste Zielstruktur wird IgM (Immunglobolin M) und als eine zweite Zielstruktur IgD (Immunglobolin D) bereitgestellt.

Außerdem werden eine Vielzahl von Festkörperpartikeln bereitgestellt, nämlich Mikrosphären aus Polystyrol mit einem Durchmesser von 6 $\mu$m. Die Festkörperpartikel stimmen bezüglich ihrer Form, ihrer Größe und ihres Materials überein.

Die Festkörperpartikel sind an ihrer Oberfläche mit daran bindenden Carboxylgruppen (-COOH) beschichtet. Anstelle der Carboxylgruppen oder zusätzlich zu diesen können an der Oberfläche der Festkörperpartikel $NH_2$-Gruppen vorgesehen sein. Über die Carboxylgruppen und/oder die $NH_2$-Gruppen können die ersten und zweiten Zielstrukturen an die Oberfläche der Festkörperpartikel gebunden werden.

Fernen werden erste und zweite Markierungsmoleküle bereitgestellt. Bei den ersten Markierungsmolekülen handelt es sich um Antigene, die für die erste Zielstruktur IgM bindungsspezifisch sind. Die zweiten Markierungsmoleküle sind Antigene, die für die zweite Zielstruktur IgD bindungsspezifisch sind. Die Markierungsmoleküle haben jeweils mindestens einen optischen Marker, der bei Anregung mit einer geeigneten Anregungsstrahlung eine Fluoreszenzstrahlung aussendet.

### 1. Kopplung von krankheitsassoziierten Antigenen an die Oberfläche der Festkörperpartikel

Es werden dreizehn Festkörperpartikel-Populationen bereitgestellt, die jeweils die gleiche Anzahl von Festkörperpartikeln umfassen, die an ihren Oberflächen Karboxylgruppen tragen.

An die Oberfläche der Festkörper-Partikel von sechs Festkörperpartikel-Populationen werden die ersten Zielstrukturen und an die Oberfläche der Festkörper-Partikel von sechs weiteren Festkörperpartikel-Populationen die zweiten Zielstrukturen gekoppelt. Hierzu werden zu den Festkörperpartikeln

- einer ersten Festkörperpartikel-Population 2,00 $\mu$g IgM,
- einer zweiten Festkörperpartikel-Population 1,00 $\mu$g IgM,
- einer dritten Festkörperpartikel-Population 0,40 $\mu$g IgM,
- einer vierten Festkörperpartikel-Population 0,20 $\mu$g IgM,
- einer fünften Festkörperpartikel-Population 0,08 $\mu$g IgM,
- einer sechsten Festkörperpartikel-Population 0,04 $\mu$g IgM,
- einer siebten Festkörperpartikel-Population 2,00 $\mu$g IgD,
- einer achten Festkörperpartikel-Population 1,00 $\mu$g IgD,
- einer neunten Festkörperpartikel-Population 0,40 $\mu$g IgD,
- einer zehnten Festkörperpartikel-Population 0,20 $\mu$g IgD,
- einer elften Festkörperpartikel-Population 0,08 $\mu$g IgD und
- einer zwölften Festkörperpartikel-Population 0,04 $\mu$g IgD,

jeweils je pro $1,2 \cdot 10^6$ Festkörperpartikel zu den Festkörperpartikeln der betreffenden Festkörperpartikel-Population dazugegeben und in einem Bindungspuffer (50mM MES, pH 5,2; 0,05% Proclin) für 60 Minuten unter ständiger Bewegung inkubiert. Die Kopplung erfolgt jeweils nach einem Standardprotokoll mittels Aktivierung durch EDAC (Carbodiimid). Die Festkörperpartikel werden gewaschen und in EDAC-Lösung (20 mg/ml) aktiviert.

Nach Zentrifugation der einzelnen Festkörperpartikel-Populationen bei 800 g wird jeweils der Überstand abgenommen und für eine Kontrollmessung der verbliebenen Antigenkonzentration zurückgestellt. Die Festkörperpartikel werden in einen Waschpuffer aufgenommen und resuspendiert. Nach nochmaliger Zentrifugation wird der Überstand verworfen und die Partikel zur Sättigung nicht besetzter reaktiver Gruppen in einen Wasch- und Storagepuffer (10mM Tris, pH 8.0; 0,05% BSA; 0,05% Proclin) aufgenommen.

An die Oberfläche der Festkörperpartikel einer dreizehnten Festkörperpartikel-Population werden weder erste noch zweite Zielstrukturen gekoppelt. Stattdessen werden die reaktiven COOH-Gruppen derart mit einem Blockierungsprotein in Kontakt gebracht, dass das Blockierungsprotein unspezifisch an die COOH-Gruppen binden kann. Als Blockierungsprotein kann beispielsweise bovines Serumalbumin oder hydrolisiertes Casein verwendet werden.

### 2. Bestimmung der für die Kopplung einzusetzenden Konzentrationen von IgM und IgD.

Jede der dreizehn Festkörperpartikel-Populationen wird jeweils mit Hilfe eines Durchflusszytometers (FACS) vermessen. Hierzu werden die Festkörperpartikel der ersten bis sechsten Festkörperpartikel-Population und die ersten Markierungsmoleküle (ca. 0,5 $\mu$g pro Messung) in einem Fluid suspendiert und für 10 Minuten im Dunkeln inkubiert. Die Proben werden zentrifugiert und der Überstand verworfen. Es wird einmal mit 2 ml PBS-Puffer (Phosphate buffered saline) gewaschen und anschließend noch einmal zentrifugiert. Nachdem der Überstand verworfen wurde, werden die Festkörperpartikel in 100-200 $\mu$l PBS aufgenommen. Die so erhaltene Suspension wird derart durch eine einen Messbereich begrenzende Düsenöffnung eines Durchflusszytometers hindurch geleitet, dass die in dem PBS enthaltenen Festkörperpartikel einzeln in einen Messbereich eines Laserstrahls gelangen. Durch den Laserstrahl werden die spezifisch an die erste Zielstruktur gebundenen ersten Markierungsmoleküle zur Aussendung einer Fluoreszenzstrahlung angeregt. Von dieser werden mit Hilfe eines optischen Detektors Fluoreszenzmesswerte erfasst. Dabei werden für jede Festkörperpartikel-Population jeweils eine Vielzahl von Fluoreszenzmesswerten (Intensitätsmesswerten) gemessen. Aus den für die einzelnen Festkörperpartikel erfassten Fluoreszenzmesswerten jede Festkörperpartikel-Population wird jeweils ein Mittelwert gebildet (MFI-Wert bzw. Median-Fluoreszenz-Intensity).

In entsprechender Weise werden die Festkörperpartikel der siebten bis zwölften Festkörperpartikel-Population mit Hilfe des Durchflusszytometers vermessen. Dabei werden durch den Laserstrahl die spezifisch an die zweite Zielstruktur gebundenen zweiten Markierungsmoleküle zur Aussendung einer Fluoreszenzstrahlung angeregt.

Obwohl humanes IgM (Immunglobolin M) aus dem Serum leicht zugänglich ist, stellte sich heraus, dass für die Kopplung an die Festkörperpartikel und insbesondere für die Stabilität nach der Kopplung, humanes rekombinant erstelltes monomeres IgM besser geeignet ist. Im Serum liegt IgM meist in einem Pentamer vor. Es zeigte sich, dass es bei der Bindung von IgM-Pentameren meist nicht zu einer vollständigen Bindung kommt. Ein Teil der IgM-Moleküle der Pentamere werden nicht kovalent an die Partikel gebunden. Die unkontrollierte Degradierung führt dann bei der späteren Verwendung zu einem Verlust der Auflösung im Durchflusszytometer. Daher werden sowohl für IgM als auch IgD (IgD (Immunglobolin D) rekombinant hergestelltes monomeres IgM und IgD verwendet.

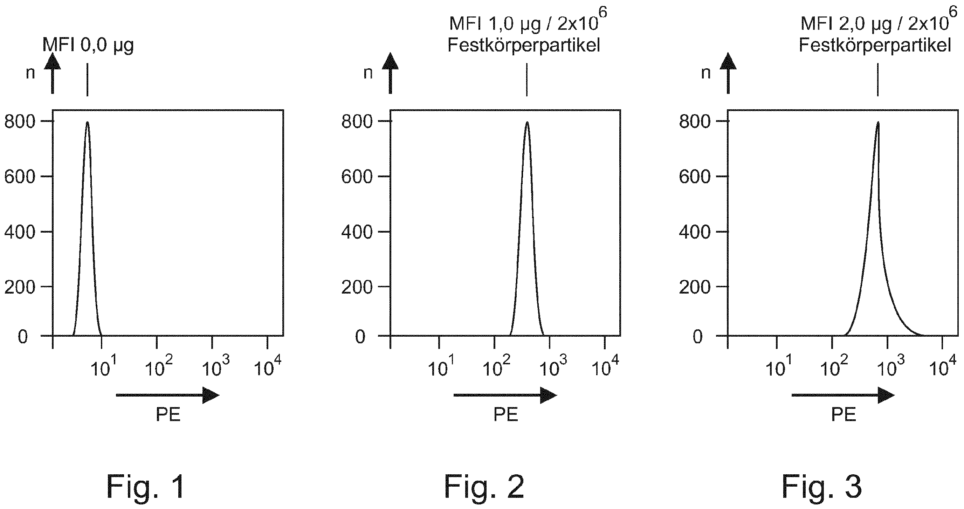

In Fig. 1 bis 3 sind die Fluoreszenzmesswerte und der Mittelwert für die erste, zweite und siebte Festkörperpartikel-Population graphisch dargestellt. Auf der Abszisse ist jeweils die mit Hilfe des Durchflusszytometers für die Festkörperpartikel der betreffende Festkörperpartikel-Population gemessene Intensität PE der Fluoreszenzstrahlung und auf der Abszisse die Anzahl n der Bindungsereignisse aufgetragen.

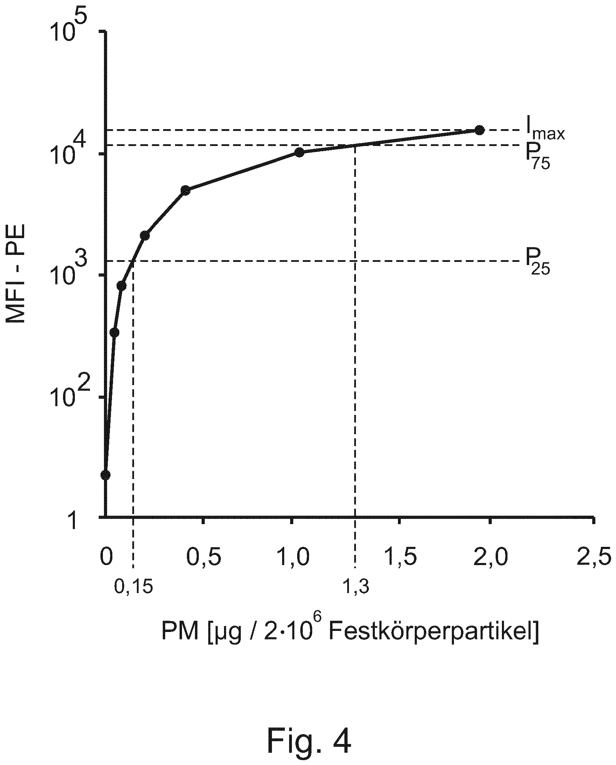

Wie in Fig. 4 zu sehen ist, werden die Mittelwerte für die einzelnen Fluoreszenzen zu einer Kopplungskurve gegen die CD23-Proteinmenge PM pro $2{,}1 \cdot 10^6$ Festkörperpartikel jeweils für die erste aufgetragen. Eine entsprechende Kopplungskurve wird für die zweite Zielstruktur erstellt.

Anhand der betreffenden Kopplungskurve kann die für eine gewünschte Intensität bzw. Signalstärke der Fluoreszenz benötigte Menge der ersten bzw. zweiten Zielstruktur abgelesen werden. Anhand der Kopplungskurve wird maximale Mittelwert der Intensität $I_{max}$ für die jeweilige Zielstruktur bestimmt und definiert. Anschließend werden ein 75%-Wert ($P_{75}$) und ein 25%-Wert ($P_{25}$) wie folgt definiert:

$$P_{75} = I_{max} \cdot 0{,}75$$

$$P_{25} = P_{75} / 10$$

Für die Kopplung ergeben sich bei dem Ausführungsbeispiel folgende Werte:

$$I_{max} = 21700$$

$$P_{75} = 21700 \cdot 0{,}75 = 16275$$

$$P_{25} = 16275 / 10 = 1627$$

Diese Werte werden als Referenz festgelegt. Aus der Kopplungskurve kann nun die passende Proteinmenge ausgelesen werden. Für $P_{75}$ beträgt diese 1,3 $\mu$g pro $2{,}1 \cdot 10^6$ Festkörperpartikel.

## 3. Zubereitung von Kalibratoren für zwei CLL-charakteristische Parameter: IgM und IgD

Ziel ist es für die ersten und zweiten Zielstrukturen (IgM und IgD) jeweils mehrere Kalibratorpopulationen zu erstellen.

Eine erste Kalibratorpopulation enthält eine Vielzahl von übereinstimmenden ersten Kalibratoren einer ersten Kalibratorart. Diese Kalibratoren haben jeweils einen Festkörperpartikel, auf dessen Oberfläche als erste Zielstruktur IgM derart immobilisiert ist, dass das Fluoreszenzsignal der einzelnen ersten Kalibratoren der ersten Kalibratorart in einer durchflusszytometrischen Analyse jeweils eine relative Intensität von 25% erreicht.

Eine zweite Kalibratorpopulation enthält eine Vielzahl von übereinstimmenden ersten Kalibratoren einer zweiten Kalibratorart. Diese Kalibratoren haben jeweils die gleichen Festkörperpartikel wir die ersten Kalibratoren der ersten Kalibratorart. Auf der Oberfläche der Festkörperpartikel der ersten Kalibratoren der zweiten Kalibratorart ist die gleiche Zielstruktur immobilisiert wie an der Oberfläche der ersten Kalibratoren der ersten Kalibratorart. Die Flächenbelegungsdichte und die Anordnung der Zielstruktur der ersten Kalibratoren der zweiten Kalibratorart ist jedoch derart gewählt, dass das Fluoreszenzsignal die ersten Kalibratoren der zweiten Kalibratorart in der durchflusszytometrischen Analyse eine relative Intensität von 75% erreicht.

Eine dritte Kalibratorpopulation enthält eine Vielzahl von übereinstimmenden ersten Kalibratoren der ersten Kalibratorart. Diese Kalibratoren haben jeweils die gleichen Festkörperpartikel wie die ersten Kalibratoren der ersten und zweiten Kalibratorpopulation. Auf den ersten Kalibratoren der der dritten Kalibratorpopulation ist eine für IgD bindungsspezifische zweite Zielstruktur derart immobilisiert, dass das Fluoreszenzsignal dier ersten Kalibratoren der ersten Kalibratorart der dritten Kalibratorpopulation in der durchflusszytometrischen Analyse eine relative Intensität von 25% erreicht.

Eine vierte Kalibratorpopulation enthält eine Vielzahl von übereinstimmenden ersten Kalibratoren der zweiten Kalibratorart. Diese Kalibratoren haben jeweils die gleichen Festkörperpartikel wie die ersten Kalibratoren der ersten, zweiten und dritten Kalibratorpopulation. Auf der Oberfläche der Festkörperpartikel der ersten Kalibratoren der vierten Kalibratorpopulation ist die gleiche Zielstruktur immobilisiert wie an der Oberfläche der ersten Kalibratoren der dritten Kalibratorpopulation. Die Flächenbelegungsdichte und die Anordnung der Zielstruktur der ersten Kalibratoren der Kalibratorpopulation ist jedoch derart gewählt, dass das Fluoreszenzsignal die ersten Kalibratoren der vierten Kalibratorpopulation in der durchflusszytometrischen Analyse eine relative Intensität von 75% erreicht.

Außerdem wird eine fünfte Kalibratorpopulation erstellt, die eine Vielzahl von übereinstimmenden ersten Kalibratoren der zweiten Kalibratorart enthält. Diese Kalibratoren haben jeweils die gleichen Festkörperpartikel wie ersten Kalibratoren der ersten, zweiten, dritten und vierten Kalibratorpopulation. Auf der Oberfläche der Festkörperpartikel der ersten Kalibratoren der fünften Kalibratorpopulation sind zwei unterschiedliche Zielstrukturen immobilisiert. Eine dieser Zielstrukturen ist mit der ersten Zielstruktur (bindungsspezifisch für IgM) und die andere Zielstruktur ist mit der zweiten Zielstruktur (für IgD bindungsspezifisches Antigen) identisch. Die Flächenbelegungsdichte und die Anordnung der Antigene IgM und IgD der ersten Kalibratoren der fünften Kalibratorpopulation ist so gewählt, dass das Fluoreszenzsignal die ersten Kalibratoren der fünften Kalibratorpopulation in der durchflusszytometrischen Analyse sowohl für IgM als auch für IgD jeweils eine relative Intensität von 75% erreicht.

Zusätzlich wird eine Negativpopulation erstellt, die eine Vielzahl von übereinstimmenden zweiten Kalibratoren aufweist. Diese bestehen jeweils aus einem Festkörperpartikel, auf dem weder die erste (IgM) noch die zweite Zielstruktur (IgD) immobilisiert ist. Bei der Negativpopulation sind die an der Oberfläche der Festkörperpartikel befindlichen Carboxylgruppen (-COOH) bzw. $NH_2$-Gruppen unspezifisch an ein Blockierungsprotein gebunden. Die Festkörperpartikel der zweiten Kalibratoren sind mit den Festkörperpartikel der ersten Kalibratoren identisch.

Für die Kopplung der Antigene an den Festkörperpartikeln wird die für jede zu erstellende Population benötigte Menge Festkörperpartikel in ein Reaktionsgefäß überführt. Die Festkörperpartikel werden bei 800 g für 5 Minuten zentrifugiert und der Überstand wird verworfen. Anschließend werden die Festkörperpartikel in einen Kopplungspuffer aufgenommen. Die Kopplung erfolgt nach dem zuvor beschriebenen Protokoll, wobei für die erste, zweite und fünfte Kalibratorpopulation eine adäquate Menge IgM und für die dritte, vierte und fünfte Kalibratorpopulation eine adäquate Menge IgD eingesetzt wird.

Die auf diese Weise bereitgestellten Kalibratorpopulationen sind nachstehend aufgelistet:

**Erste Kalibratoren:**

| | |
|---|---|
| a) IgM-Kalibratoren: | $PD_{25}$ - IgM Konzentration $Int_{25}$ |
| | PD75 - IgM Konzentration $Int_{75}$ |
| b) IgD-Kalibratoren: | $PD_{25}$ - IgD Konzentration $Int_{25}$ |
| | $PD_{75}$ - IgD Konzentration $Int_{75}$ |
| c) IgM- und IgD- Kalibratoren: | $P_{md}$ - IgM und IgD Konzentration $Int_{75}$ |
| **Zweite Kalibratoren:** | $P_0$ - weder IgM noch IgD |

Die einzelnen Kalibratoren werden nach ihrer Herstellung mittels FACS (fluorescence-activated cell sorting) unter Verwendung kommerziell erhältlicher, mit einem Marker markierter Antikörper vermessen. Für eine Anwendung zum Zwecke der späteren Diagnostik werden die Kalibratoren zu einem Kit zusammengefasst. So wird bei der späteren FACS-Messung, zu diagnostischen Zwecken, der Messraum durch die einzelnen Populationen definiert, standardisiert und normiert.

**4. Zubereitung von Kalibratoren mit drei CLL-charakteristischen Parametern IgM, IgD und CD19**

Bei einem zweiten Ausführungsbeispiel werden für die Verwendung weiterer Parameter die Anzahl und die Kombination der einzelnen Populationen gegenüber dem ersten Ausführungsbeispiel erweitert. Wie für IgM und IgD beschrieben, wurde auch für das humane und rekombinant erstellte B-Lymphocyt Antigen CD19 zunächst eine Standardkurve für die effektive Intensität erstellt. Im Bezug darauf wurden die entsprechenden Mengen für die Kopplung verwendet.

**Erste Kalibratoren:**

| | |
|---|---|
| a) IgM-Kalibratoren: | $PD_{25}$ - IgM Konzentration $Int_{25}$ |
| | PD75 - IgM Konzentration $Int_{75}$ |
| b) IgD-Kalibratoren: | $PD_{25}$ - IgD Konzentration $Int_{25}$ |
| | PD75 - IgD Konzentration $Int_{75}$ |
| c) CD19-Kalibratoren: | $PD_{25}$ - CD19 Konzentration $Int_{25}$ |
| | $PD_{75}$ - CD19 Konzentration $Int_{75}$ |
| d) IgM- und IgD- Kalibratoren: | $P_{md}$ - IgM und IgD Konzentration $Int_{75}$ |
| e) CD19- und IgM-Kalibratoren: | $P_{CD19/m}$ - CD19 und IgM Konzentration $Int_{75}$ |
| f) CD19- und IgD-Kalibratoren: | $P_{CD19/d}$ - CD19 und IgD Konzentration $Int_{75}$ |
| **Zweite Kalibratoren:** | $P_0$ - weder IgM noch IgD noch CD19 |

Dieses Verfahren lässt sich auf eine beliebige Anzahl von Parametern erweitern und variieren.

**5. Verwendung der Kalibratoren zur Analyse von Blutproben mittels FACS**

Es wird ein Kit bereitgestellt, der folgendes aufweist:

a) Eine erste Population von ersten Kalibratoren der ersten Kalibratorart, die eine Vielzahl der in Abschnitt 3 beschriebenen ersten Kalibratoren $PD_{25}$ umfasst, an deren Oberfläche als erste Zielstruktur IgM immobilisiert ist.
b) Eine zweite Population von ersten Kalibratoren zweiten Kalibratorart, die eine Vielzahl der in Abschnitt 3 beschriebenen ersten Kalibratoren $PD_{75}$ umfasst, an deren Oberfläche als erste Zielstruktur IgM immobilisiert ist.
c) Eine erste Population von ersten Kalibratoren der ersten Kalibratorart, die eine Vielzahl der in Abschnitt 3 beschriebenen ersten Kalibratoren $PD_{25}$ umfasst, an deren Oberfläche als erste Zielstruktur IgD immobilisiert ist.
d) Eine zweite Population von ersten Kalibratoren zweiten Kalibratorart, die eine Vielzahl der in Abschnitt 3 beschriebenen ersten Kalibratoren $PD_{75}$ umfasst, an deren Oberfläche als erste Zielstruktur IgD immobilisiert ist.
e) Eine Population von zweiten Kalibratoren, die eine Vielzahl der in Abschnitt 3 beschriebenen zweiten Kalibratoren $P_0$ umfasst.
f) Eine Population von ersten Markierungsmolekülen, die jeweils ein erstes Antigen aufweisen, welches für die erste Zielstruktur IgM bindungsspezifisch ist. Die ersten Markierungsmoleküle weisen jeweils einen ersten optischen Marker auf, der an das erste Antigen gekoppelt ist und bei Anregung mit einer geeigneten ersten Anregungsstrahlung eine erste Fluoreszenzstrahlung aussendet.
g) Eine Population von zweiten Markierungsmolekülen, die jeweils ein zweites Antigen aufweisen, welches für die zweite Zielstruktur IgD bindungsspezifisch ist. Die zweiten Markierungsmoleküle weisen jeweils einen zweiten optischen Marker auf, der an das zweite Antigen gekoppelt ist und bei Anregung mit einer geeigneten zweiten Anregungsstrahlung eine zweite Fluoreszenzstrahlung aussendet.
h) eine Phosphat-gepufferte Salzlösung (PBS-Puffer).

Bei einer FACS-basierten Analyse von Blutzellen wird nach der Blutentnahme die Zellzahl der zu klassifizierenden Zellen (Lymphomzellen) im Analysemedium Blut bestimmt und die benötigte Zellzahl für jede FACS-Analyse in ein Reaktionsgefäß gegeben (z.B. $1 \times 10^6$ Zellen pro Messung). Anschließend werden die Markierungsmoleküle (ca. 0,5 μg pro Messung) und die Kalibratoren $P_0$, $PD_{25}$, $PD_{75}$ hinzugefügt und für 10 Minuten im Dunkeln inkubiert. Nach der Inkubationszeit wird das Volumen mit 2 ml Lyse-Fixierungspuffer aufgefüllt und noch einmal für 10 Minuten inkubiert. In diesem Schritt werden die Zellen nicht nur fixiert, sondern die Mehrheit der roten Blutkörperchen zum Platzen gebracht. Diese sind für die Messung nicht von Bedeutung und würden diese unter Umständen stören.

Die Proben werden zentrifugiert und der Überstand verworfen. Es wird einmal mit 2 ml PBS-Puffer (Phosphate buffered saline) gewaschen und anschließend noch einmal zentrifugiert. Nachdem der Überstand verworfen wurde, werden die Zellen in 100-200 μl PBS aufgenommen.

Das so erhaltene Analysemedium wird anschließend in einem FACS-Durchflusszytometer analysiert. Im FACS-Durchflusszytometer wird das Analysemedium derart durch eine einen Messbereich begrenzende Düsenöffnung hindurch geleitet, dass sowohl die mit dem Markierungsmolekül markierten Zellen als auch die unterschiedlichen Kalibratoren jeweils einzeln in den Messbereich von Laserstrahlen gelangen.

Mit Hilfe des FACS-Durchflusszytometers werden für jeden in den Messbereich gelangenden Kalibrator und für jede in den Messbereich gelangende Zelle jeweils ein erster und zwei zweite Durchflusszytometrie-Messwerte erfasst. Der erste Durchflusszytometrie-Messwert ist von der Fluoreszenzstrahlung abhängig, welche mindestens ein an den betreffenden, im Messbereich befindlichen Kalibrator bzw. die betreffende, im Messbereich befindliche Zelle gebundenes Markierungsmolekül aufgrund der Anregung durch den Laserstrahl abgibt. Die beiden zweiten Durchflusszytometrie-Messwerte umfassen einen Vorwärtsstreuungs-messwert und einen Seitwärtsstreuungsmesswert für die beim Auftreffen des Laserstrahls auf den betreffenden, im Messbereich befindlichen Kalibrator bzw. die betreffende, im Messbereich befindliche Zelle erzeugte Streustrahlung.

Die zweiten Durchflusszytometrie-Messwerte werden mit Referenzwerten verglichen. In Abhängigkeit vom Ergebnis des Vergleichs lässt sich aufgrund der unterschiedlichen Streuungseigenschaften von Zellen und Kalibratoren feststellen, ob die zweiten Durchflusszytometrie-Messwerte durch Streuung des Laserstrahls an einer Zelle oder an einem Kalibrator verursacht wurden. Die unterschiedlichen Kalibratoren lassen sich anhand der Signalpegel ihrer ersten Durchflusszytometrie-Messwerte unterscheiden. Demgemäß wird in Abhängigkeit vom Ergebnis des Vergleichs der erste Durchflusszytometrie-Messwerte entweder den Zellen oder einem Kalibrator zugeordnet.

Falls es sich um einen Kalibrator handelt wird der erste Durchflusszytometrie-Messwert mit Vergleichsintervallen verglichen, welche des unterschiedlichen Kalibratorpartikel-Populationen zugeordnet sind. Somit lässt sich erste Durchflusszytometrie-Messwert einem Kalibrator einer bestimmten Kalibratorpartikel-Population zuordnen. Die Zuordnung kann automatisiert erfolgen, beispielsweise mittels einer geeigneten Software.

Für jede einzelne Zielstruktur (IgM bzw. IgD) werden für die einzelnen Kalibratoren $P_0$, $PD_{25}$ und $PD_{75}$ die jeweiligen Mittelwerte der ersten Durchflusszytometrie-Messwerte (Fluoreszenz-Intensitäten) bestimmt. Diese bilden die Grundlage für den zur Messung genutzten Referenz-Messraum. Der erste Durchflusszytometrie-Messwert für die Kalibratoren $P_0$ dient dazu, den Hintergrund in dem Kanal zu bestimmen. Außerdem ermöglicht das erste Durchflusszytometrie-Messwert für die Kalibratoren $P_0$ eine Aussage über die Qualität der Kopplung der Antigene der Markierungsmoleküle an die ihnen jeweils zugeordnete Zielstruktur (Negativ-Kontrolle).

Außerdem werden für die Zellen für jede einzelne Zielstruktur Mittelwerte der ersten Durchflusszytometrie-Messwerte bestimmt.

Bei dem Ausführungsbeispiel werden für IgM folgende Mittelwerte für die ersten Durchflusszytometrie-Messwerte ermittelt:

| | |
|---|---|
| Erste Kalibratoren $PD_{25}$ der ersten Kalibratorart: | 1600 |
| Erste Kalibratoren $PD_{75}$ der zweiten Kalibratorart:: | 16000 |
| Zweite Kalibratoren $P_0$: | 25 |
| Zellen für die erste Zielstruktur: | 5600 |

Zunächst wird das Hintergrundrauschen aus den Messwerten bereinigt. Das Hintergrundrauschen entsteht durch unspezifische Bindungen der Antikörper der verwendeten Markierungsmoleküle an die Festkörperpartikel der Kalibratoren bzw. an die Zellen. Dazu wird der erste Durchflusszytometrie-Messwert der zweiten Kalibratoren $P_0$ jeweils von den ersten Durchflusszytometrie-Messwerten der ersten Kalibratoren $PD_{25}$ der ersten Kalibratorart, der ersten Kalibratoren $PD_{75}$ der zweiten Kalibratorart, der zweiten Kalibratoren $P_0$ und der Zellen subtrahiert. Es ergeben sich somit für IgM folgende bereinigte erste Durchflusszytometrie-Messwerte:

| | |
|---|---|
| Erste Kalibratoren $PD_{25}$ der ersten Kalibratorart: | 1600 - 25 = 1575 |
| Erste Kalibratoren $PD_{75}$ der zweiten Kalibratorart: | 16000 - 25 = 15975 |
| Zweite Kalibratoren $P_0$: | 25 - 25 = 0 |

(fortgesetzt)

| Zellen für die erste Zielstruktur: | 5600 - 25 = 5575. |
|---|---|

Für die Kalibratoren $PD_{25}$ und $PD_{75}$ werden Referenzeinheiten definiert. Dem bereinigten ersten Durchflusszytometrie-Messwert des Kalibrators $PD_{75}$ der zweiten Kalibratorart werden 100 Referenzeinheiten zugewiesen. Somit entspricht eine Referenzeinheit einem bereinigten ersten Durchflusszytometrie-Messwert von 15975 / 100 = 159,75. Diese Zahl wird nachstehend auch als erster Skalierungsfaktor bezeichnet. Der bereinigte erste Durchflusszytometrie-Messwert des Kalibrators $PD_{25}$ der ersten Kalibratorart entspricht 1575 / 159,75 = 9,86 Referenzeinheiten. Aus dem bereinigten ersten Durchflusszytometrie-Messwert für die erste Zielstruktur der Zellen und dem ersten Skalierungsfaktor wird ein erster normierter Messwert gebildet: 5575 / 159,75 = 34,90 Referenzeinheiten.

Bei dem Ausführungsbeispiel werden für IgD folgende Mittelwerte für die ersten Durchflusszytometrie-Messwerte ermittelt:

| Erste Kalibratoren $PD_{25}$ der ersten Kalibratorart: | 3103 |
|---|---|
| Erste Kalibratoren $PD_{75}$ der zweiten Kalibratorart:: | 31030 |
| Zweite Kalibratoren $P_0$: | 144 |
| Zellen für die zweite Zielstruktur: | 4450 |

Es ergeben sich somit für IgD folgende bereinigte erste Durchflusszytometrie-Messwerte:

| Erste Kalibratoren $PD_{25}$ der ersten Kalibratorart: | 3103 - 144 = 2959 |
|---|---|
| Erste Kalibratoren $PD_{75}$ der zweiten Kalibratorart: | 31030 - 144 = 30886 |
| Zweite Kalibratoren $P_0$: | 144 - 144 = 0 |
| Zellen für die zweite Zielstruktur: | 4450 - 144 = 4306. |

Für die Kalibratoren $PD_{25}$ und $PD_{75}$ werden wiederum Referenzeinheiten definiert. Dem bereinigten ersten Durchflusszytometrie-Messwert des Kalibrators $PD_{75}$ der zweiten Kalibratorart werden 100 Referenzeinheiten zugewiesen. Somit entspricht eine Referenzeinheit einem bereinigten ersten Durchflusszytometrie-Messwert von 30886 / 100 = 308,86. Diese Zahl wird nachstehend auch als zweiter Skalierungsfaktor bezeichnet. Der bereinigte erste Durchflusszytometrie-Messwert des Kalibrators $PD_{25}$ der ersten Kalibratorart entspricht 2959 / 308,86 = 9,58 Referenzeinheiten. Aus dem bereinigten ersten Durchflusszytometrie-Messwert für die zweite Zielstruktur der Zellen und dem zweiten Skalierungsfaktor wird ein zweiter normierter Messwert gebildet: 4306 / 308,86 = 13,94 Referenzeinheiten.

Durch die Zuweisung der Referenzeinheiten werden die bereinigten ersten Durchflusszytometrie-Messwerte der Zellen von den Eigenschaften und Einstellungen des verwendeten Durchflusszytometers unabhängig und können mit entsprechenden bereinigten ersten Durchflusszytometrie-Messwerte, die mit einem Durchflusszytometer, das anders eingestellt ist als das zuerst genannte Durchflusszytometer oder das andere Eigenschaften aufweist als dieses verglichen werden.

In einem späteren Schritt können bei Bedarf die auf die Referenzeinheiten bezogenen normierten Messwerte der Zellen, mit denen der Kopplungskurve (Fig. 4) in Bezug gesetzt werden. Die Kopplungskurve entspricht einer logistischen Funktion (Gauß-Lorentz Verteilung). Mit Hilfe einer "Best-Fitting"-Kurve können so aus den in Referenzeinheiten angegebenen Werten Absolutwerte bestimmt werden, die der Menge der betreffenden, auf den Zellen befindlichen Zielstruktur entsprechen.

**Patentansprüche**

1. Durchflusszytometrie-Messverfahren, bei dem ein Analysemedium bereitgestellt wird, welches ein Fluid und darin enthaltene, zu klassifizierende biologische Zellen aufweist,

   - wobei mindestens ein Markierungsmolekül bereitgestellt und derart mit dem Analysemedium in Kontakt gebracht wird, dass das Markierungsmolekül spezifisch an mindestens eine an der Oberfläche der Zellen befindliche Zielstruktur binden kann, wenn die Zelle diese Zielstruktur aufweist,
   - wobei ein Fluidstrom des Analysemediums erzeugt wird, in dem die Zellen einzeln in einen Messbereich eines Energiestrahls und/oder eines elektrischen Felds gelangen,
   - wobei für die einzelnen, in dem Messbereich befindlichen Zellen jeweils zumindest für einen ersten physikalischen Parameter ein erster und für einen zweiten physikalischen Parameter ein zweiter Durchflusszytometrie-Messwert erfasst werden,

- wobei zumindest der erste Parameter eine Fluoreszenzstrahlung ist, welche das mindestens eine Markierungsmolekül bei Anregung mit dem Energiestrahl oder dem elektrischen Feld abgibt,

- wobei die Zellen anhand der Durchflusszytometrie-Messwerte klassifiziert werden,

- wobei mindestens zwei Kalibratorarten von ersten Kalibratoren und mindestens ein zweiter Kalibrator bereitgestellt werden,

- wobei die ersten Kalibratoren und der mindestens eine zweite Kalibrator jeweils einen Festkörperpartikel aus einem nicht wasserlöslichen, anorganischen und/oder polymeren Material aufweisen und die Festkörperpartikel der ersten Kalibratoren und des mindestens einen zweiten Kalibrators bezüglich ihrer Form, ihrer Größe und ihres Materials übereinstimmen,

- wobei die ersten Kalibratoren an ihrer Oberfläche mindestens eine Zielstruktur aufweisen, die mit der mindestens einen Zielstruktur der Zellen übereinstimmt und auf den Festkörperpartikeln der ersten Kalibratoren immobilisiert ist,

- wobei der mindestens eine zweite Kalibrator diese Zielstruktur nicht aufweist,

- wobei die ersten Kalibratoren und der mindestens eine zweite Kalibrator mit dem Analysemedium vermischt werden, bevor die Durchflusszytometrie-Messwerte erfasst werden, derart, dass mindestens ein in dem Analysemedium befindliches Markierungsmolekül an die mindestens eine Zielstruktur der ersten Kalibratoren bindet,

- wobei die Kalibratoren in dem Fluidstrom einzeln nacheinander in den Messbereich eingebracht werden,

- wobei für die ersten Kalibratoren und den mindestens einen zweiten Kalibrator entsprechende erste und zweite Durchflusszytometrie-Messwerte erfasst werden wie für die Zellen,

- wobei der dem zweiten Durchflusszytometrie-Messwert der Zellen und dem zweiten Durchflusszytometrie-Messwert der Kalibratoren zugeordnete Parameter derart gewählt wird, dass sich die Kalibratoren anhand der zweiten Durchflusszytometrie-Messwerte von den Zellen unterscheiden lassen,

- wobei sich die Kalibratoren der unterschiedlichen Kalibratorarten insbesondere hinsichtlich der Flächenbelegungsdichte und/oder der Anordnung ihrer mindestens einen Zielstruktur auf der Oberfläche des Festkörperpartikels derart voneinander unterscheiden, dass die ersten Durchflusszytometrie-Messwerte einer ersten Kalibratorart eine Signalstärke aufweisen, die größer ist als die Signalstärke der ersten Durchflusszytometrie-Messwerte der zweiten Kalibratoren und kleiner ist als die Signalstärke der ersten Durchflusszytometrie-Messwerte einer zweiten Kalibratorart,

- wobei zur Bildung eines normalisierten ersten Durchflusszytometrie-Messwerts für die erste Kalibratorart die Differenz zwischen dem ersten Durchflusszytometrie-Messwert des ersten Kalibrators der ersten Kalibratorart und dem ersten Durchflusszytometrie-Messwert des zweiten Kalibrators zur Differenz zwischen dem ersten Durchflusszytometrie-Messwert der Zelle und dem ersten Durchflusszytometrie-Messwert des zweiten Kalibrators ins Verhältnis gesetzt wird, und

- wobei zur Bildung eines normalisierten ersten Durchflusszytometrie-Messwerts für die zweite Kalibratorart die Differenz zwischen dem ersten Durchflusszytometrie-Messwert des ersten Kalibrators der zweiten Kalibratorart und dem ersten Durchflusszytometrie-Messwert des zweiten Kalibrators zur Differenz zwischen dem ersten Durchflusszytometrie-Messwert der Zelle und dem ersten Durchflusszytometrie-Messwert des zweiten Kalibrators ins Verhältnis gesetzt wird.

2. Durchflusszytometrie-Messverfahren, bei dem ein Analysemedium bereitgestellt wird, welches ein Fluid und darin enthaltene, zu klassifizierende biologische Zellen aufweist,

- wobei mindestens ein Markierungsmolekül bereitgestellt und derart mit dem Analysemedium in Kontakt gebracht wird, dass das Markierungsmolekül spezifisch an mindestens eine an der Oberfläche der Zellen befindliche Zielstruktur binden kann, wenn die Zelle diese Zielstruktur aufweist,

- wobei ein Fluidstrom des Analysemediums erzeugt wird, in dem die Zellen einzeln in einen Messbereich eines Energiestrahls und/oder eines elektrischen Felds gelangen,

- wobei für die einzelnen, in dem Messbereich befindlichen Zellen jeweils zumindest für einen ersten physikalischen Parameter ein erster und für einen zweiten physikalischen Parameter ein zweiter Durchflusszytometrie-Messwert erfasst werden,

- wobei zumindest der erste Parameter eine Fluoreszenzstrahlung ist, welche das mindestens eine Markierungsmolekül bei Anregung mit dem Energiestrahl oder dem elektrischen Feld abgibt,

- wobei die Zellen anhand der Durchflusszytometrie-Messwerte klassifiziert werden,

- wobei mindestens zwei Kalibratorarten, die jeweils mehrere identische erste Kalibratoren aufweisen, und mehrere identische zweite Kalibratoren bereitgestellt werden,

- wobei die ersten und zweiten Kalibratoren jeweils einen Festkörper-partikel aus einem nicht wasserlöslichen, anorganischen und/oder polymeren Material aufweisen und die Festkörperpartikel der ersten und zweiten Kalibratoren bezüglich ihrer Form, ihrer Größe und ihres Materials übereinstimmen,

- wobei die ersten Kalibratoren an ihrer Oberfläche mindestens eine Zielstruktur aufweisen, die mit der mindestens einen Zielstruktur der Zellen übereinstimmt und auf den Festkörperpartikeln der ersten Kalibratoren immobilisiert ist,

- wobei die zweiten Kalibratoren diese Zielstruktur nicht aufweisen,

- wobei die ersten und zweiten Kalibratoren mit dem Analysemedium vermischt werden, bevor die Durchflusszytometrie-Messwerte erfasst werden, derart, dass mindestens ein in dem Analysemedium befindliches Markierungsmolekül an die mindestens eine Zielstruktur der ersten Kalibratoren bindet,

- wobei die Kalibratoren in dem Fluidstrom einzeln nacheinander in den Messbereich eingebracht werden,

- wobei für die ersten und zweiten Kalibrator entsprechende erste und zweite Durchflusszytometrie-Messwerte erfasst werden wie für die Zellen,

- wobei aus den ersten Durchflusszytometrie-Messwerten der ersten Kalibratoren jeder Kalibratorart jeweils ein gemittelter erster Durchflusszytometrie-Messwert für die ersten Kalibratoren der betreffenden Kalibratorart und aus den ersten Durchflusszytometrie-Messwerten der zweiten Kalibratoren ein gemittelter erster Durchflusszytometrie-Messwert für die zweiten Kalibratoren gebildet wird,

- wobei der dem zweiten Durchflusszytometrie-Messwert der Zellen und dem zweiten Durchflusszytometrie-Messwert der Kalibratoren zugeordnete Parameter derart gewählt wird, dass sich die Kalibratoren anhand der zweiten Durchflusszytometrie-Messwerte von den Zellen unterscheiden lassen,

- wobei sich die Kalibratoren der unterschiedlichen Kalibratorarten insbesondere hinsichtlich der Flächenbelegungsdichte und/oder der Anordnung ihrer mindestens einen Zielstruktur auf der Oberfläche des Festkörperpartikels derart voneinander unterscheiden, dass die ersten Durchflusszytometrie-Messwerte einer ersten Kalibratorart eine Signalstärke aufweisen, die größer ist als die Signalstärke der ersten Durchflusszytometrie-Messwerte der zweiten Kalibratoren und kleiner ist als die Signalstärke der ersten Durchflusszytometrie-Messwerte einer zweiten Kalibratorart,

- wobei zur Bildung eines normalisierten ersten Durchflusszytometrie-Messwerts für die erste Kalibratorart die Differenz zwischen dem gemittelten ersten Durchflusszytometrie-Messwert der ersten Kalibratoren der ersten Kalibratorart und dem gemittelten ersten Durchflusszytometrie-Messwert der zweiten Kalibratoren zur Differenz zwischen dem ersten Durchflusszytometrie-Messwert der Zelle und dem gemittelten ersten Durchflusszytometrie-Messwert der zweiten Kalibratoren ins Verhältnis gesetzt wird, und

- wobei zur Bildung eines normalisierten ersten Durchflusszytometrie-Messwerts für die zweite Kalibratorart die Differenz zwischen dem gemittelten ersten Durchflusszytometrie-Messwert der ersten Kalibratoren der zweiten Kalibratorart und dem gemittelten ersten Durchflusszytometrie-Messwert der zweiten Kalibratoren zur Differenz zwischen dem ersten Durchflusszytometrie-Messwert der Zelle und dem gemittelten ersten Durchflusszytometrie-Messwert der zweiten Kalibratoren ins Verhältnis gesetzt wird.

3. Durchflusszytometrie-Messverfahren nach Anspruch 1 oder 2, **dadurch gekennzeichnet, dass** für mindestens einen ersten Kalibrator ein erster Kalibrierfaktor bereitgestellt wird, der bezüglich des Messignals für den ersten Parameter dem Verhältnis zwischen der Messsignalstärke des ersten Kalibrators und der Messsignalstärke eines die Zielstruktur aufweisenden ersten Referenz-Kalibrators entspricht, dass für den ersten physikalischen Parameter des ersten Kalibrators ein erstes Messsignal erfasst wird und der erste Durchflusszytometrie-Messwert des ersten Kalibrators aus dem ersten Messsignal und dem ersten Kalibrierfaktor gebildet wird, dass für den zweiten Kalibrator ein zweiter Kalibrierfaktor bereitgestellt wird, der bezüglich des Messignals für den ersten Parameter dem Verhältnis zwischen der Messsignalstärke des zweiten Kalibrators und der Messsignalstärke eines die Zielstruktur nicht aufweisenden zweiten Referenz-Kalibrators entspricht, und dass für den ersten physikalischen Parameter des zweiten Kalibrators ein zweites Messsignal erfasst wird und der erste Durchflusszytometrie-Messwert des zweiten Kalibrators aus dem zweiten Messsignal und dem zweiten Kalibrierfaktor gebildet wird.

4. Durchflusszytometrie-Messverfahren nach einem der Ansprüche 1 bis 3, **dadurch gekennzeichnet, dass** in dem Fluid mindestens zwei unterschiedliche Populationen von Zellen enthalten sind, die sich derart voneinander unterscheiden, dass die ersten Durchflusszytometrie-Messwerte der Zellen einer ersten Population in einem ersten Signalstärkebereich und die die ersten Durchflusszytometrie-Messwerte der Zellen einer zweiten Population in einem außerhalb des ersten Signalstärkebereichs befindlichen zweiten Signalstärkebereich liegen, und dass die Signalstärke der ersten Durchflusszytometrie-Messwerte der ersten Kalibratorart derart gewählt wird, dass sie zwischen dem ersten und zweiten Signalstärkebereich liegt.

5. Durchflusszytometrie-Messverfahren nach einem der Ansprüche 1 bis 4, **dadurch gekennzeichnet, dass** die größte Abmessung der Festkörperpartikel kleiner als 20 $\mu$m, insbesondere kleiner als 15 $\mu$m und bevorzugt kleiner als 10

μm ist und/oder dass die kleinste Abmessung der Festkörperpartikel größer als 4 μm, insbesondere größer als 5 μm und bevorzugt größer als 6 μm ist.

**6.** Durchflusszytometrie-Messverfahren nach einem der Ansprüche 1 bis 5, **dadurch gekennzeichnet, dass** das Material der Festkörperpartikel Polystyren, Melamin, Latex oder ein Silikat ist.

**7.** Durchflusszytometrie-Messverfahren nach einem der Ansprüche 1 bis 6, **dadurch gekennzeichnet, dass** der mindestens eine zweite Durchflusszytometrie-Messwert einen Vorwärtsstreuungs-Messwert für eine in dem Messbereich auftretende Vorwärtsstreuung des Energiestrahls und/oder einen Seitwärtsstreuungs-Messwert für eine in dem Messbereich auftretende Seitwärtsstreuung des Energiestrahls umfasst.

**8.** Durchflusszytometrie-Messverfahren nach einem der Ansprüche 1 bis 7, **dadurch gekennzeichnet, dass** sowohl für die Zellen als auch für die ersten und zweiten Kalibratoren jeweils für mehrere Messkanäle mindestens ein erster Durchflusszytometrie-Messwert erfasst wird, dass zumindest eine der Anzahl der Messkanäle entsprechende Anzahl von unterschiedlichen Markierungsmolekülen bereitgestellt und mit dem Analysemedium in Kontakt gebracht wird, und dass der mindestens eine erste Kalibrator zumindest eine der Anzahl der Messkanäle entsprechende Anzahl von Zielstrukturen aufweist, die jeweils spezifisch an eines der unterschiedlichen Markierungsmoleküle binden, wenn sie mit dem betreffenden Markierungsmolekül in Kontakt gelangen.

**9.** Durchflusszytometrie-Messverfahren nach einem der Ansprüche 1 bis 8, **dadurch gekennzeichnet, dass** die Zielstruktur auf den ersten Kalibratoren mindestens ein Antigen aufweist, das vorzugsweise ein mindestens ein Protein und/oder mindestens ein Peptid und/oder mindestens einen Rezeptor und/oder mindestens ein Allergen und/oder mindestens ein glykosyliertes Protein und/oder mindestens ein Liposaccharid und/oder mindestens ein Oligosacharide und/oder mindestens ein Polysaccharid und/oder mindestens eine Nukleinsäuren und/oder mindestens ein Fragmente und/oder Derivate der vorgenannten Substanzen umfasst.

**10.** Durchflusszytometrie-Messverfahren nach einem der Ansprüche 1 bis 9, **dadurch gekennzeichnet, dass** die Zielstruktur des mindestens einen ersten Kalibrators über mindestens eine aktivierte Carboxy-Gruppen und/oder mindestens eine aktivierte NH2-Gruppe am Festköperpartikel des ersten Kalibrators immobilisiert ist.

**Claims**

**1.** Flow cytometry measurement method in which there is provided an analysis medium which comprises a fluid and, contained therein, biological cells to be classified,

- wherein at least one labelling molecule is provided and is contacted with the analysis medium such that the labelling molecule can specifically bind to at least one target structure present on the surface of the cells, if the cell has said target structure,
- wherein a fluid stream of the analysis medium is generated, in which the cells individually enter a measurement region of an energy beam and/or an electric field,
- wherein, for the individual cells present in the measurement region, respective measurements are made of at least a first flow cytometry measurement value for a first physical parameter and a second flow cytometry measurement value for a second physical parameter,
- wherein at least the first parameter is a fluorescence radiation which is emitted by the at least one labelling molecule upon excitation by the energy beam or the electric field,
- wherein the cells are classified on the basis of the flow cytometry measurement values,
- wherein at least two calibrator types of first calibrators and at least one second calibrator are provided,
- wherein the first calibrators and the at least one second calibrator each comprise a solid particle composed of a non-water-soluble, inorganic and/or polymeric material and the solid particles of the first calibrators and the at least one second calibrator are identical with regard to the shape, size and material thereof,
- wherein the first calibrators have, on their surface, at least one target structure which is identical to the at least one target structure of the cells and has been immobilized on the solid particles of the first calibrators,
- wherein the at least one second calibrator does not have said target structure,
- wherein the first calibrators and the at least one second calibrator are mixed with the analysis medium before the flow cytometry measurement values are measured, such that at least one labelling molecule present in the analysis medium binds to the at least one target structure of the first calibrators,
- wherein the calibrators are individually introduced into the measurement region one after another in the fluid stream,

- wherein, for the first calibrators and the at least one second calibrator, corresponding first and second flow cytometry measurement values are measured as for the cells,
- wherein the parameter assigned to the second flow cytometry measurement value of the cells and to the second flow cytometry measurement value of the calibrators is chosen such that the calibrators can differ from the cells on the basis of the second flow cytometry measurement values,
- wherein the calibrators of the different calibrator types differ from one another especially with respect to the surface coverage density and/or the arrangement of their at least one target structure on the surface of the solid particle such that the first flow cytometry measurement values of a first calibrator type have a signal intensity greater than the signal intensity of the first flow cytometry measurement values of the second calibrators and less than the signal intensity of the first flow cytometry measurement values of a second calibrator type,
- wherein, for formation of a normalized first flow cytometry measurement value for the first calibrator type, the ratio of the difference between the first flow cytometry measurement value of the first calibrator of the first calibrator type and the first flow cytometry measurement value of the second calibrator to the difference between the first flow cytometry measurement value of the cell and the first flow cytometry measurement value of the second calibrator is formed, and
- wherein, for formation of a normalized first flow cytometry measurement value for the second calibrator type, the ratio of the difference between the first flow cytometry measurement value of the first calibrator of the second calibrator type and the first flow cytometry measurement value of the second calibrator to the difference between the first flow cytometry measurement value of the cell and the first flow cytometry measurement value of the second calibrator is formed.

2. Flow cytometry measurement method in which there is provided an analysis medium which comprises a fluid and, contained therein, biological cells to be classified,

- wherein at least one labelling molecule is provided and is contacted with the analysis medium such that the labelling molecule can specifically bind to at least one target structure present on the surface of the cells, if the cell has said target structure,
- wherein a fluid stream of the analysis medium is generated, in which the cells individually enter a measurement region of an energy beam and/or an electric field,
- wherein, for the individual cells present in the measurement region, respective measurements are made of at least a first flow cytometry measurement value for a first physical parameter and a second flow cytometry measurement value for a second physical parameter,
- wherein at least the first parameter is a fluorescence radiation which is emitted by the at least one labelling molecule upon excitation by the energy beam or the electric field,
- wherein the cells are classified on the basis of the flow cytometry measurement values,
- wherein at least two calibrator types, which each comprise multiple identical first calibrators, and multiple identical second calibrators are provided,
- wherein the first and second calibrators each comprise a solid particle composed of a non-water-soluble, inorganic and/or polymeric material and the solid particles of the first and second calibrators are identical with regard to the shape, size and material thereof,
- wherein the first calibrators have, on their surface, at least one target structure which is identical to the at least one target structure of the cells and has been immobilized on the solid particles of the first calibrators,
- wherein the second calibrators do not have said target structure,
- wherein the first and second calibrators are mixed with the analysis medium before the flow cytometry measurement values are measured, such that at least one labelling molecule present in the analysis medium binds to the at least one target structure of the first calibrators,
- wherein the calibrators are individually introduced into the measurement region one after another in the fluid stream,
- wherein, for the first and second calibrators, corresponding first and second flow cytometry measurement values are measured as for the cells,
- wherein the first flow cytometry measurement values of the first calibrators of each calibrator type are used to form, in each case, an averaged first flow cytometry measurement value for the first calibrators of the calibrator type in question and the first flow cytometry measurement values of the second calibrators are used to form an averaged first flow cytometry measurement value for the second calibrators,
- wherein the parameter assigned to the second flow cytometry measurement value of the cells and to the second flow cytometry measurement value of the calibrators is chosen such that the calibrators can differ from the cells on the basis of the second flow cytometry measurement values,

- wherein the calibrators of the different calibrator types differ from one another especially with respect to the surface coverage density and/or the arrangement of their at least one target structure on the surface of the solid particle such that the first flow cytometry measurement values of a first calibrator type have a signal intensity greater than the signal intensity of the first flow cytometry measurement values of the second calibrators and less than the signal intensity of the first flow cytometry measurement values of a second calibrator type,

- wherein, for formation of a normalized first flow cytometry measurement value for the first calibrator type, the ratio of the difference between the averaged first flow cytometry measurement value of the first calibrators of the first calibrator type and the averaged first flow cytometry measurement value of the second calibrators to the difference between the first flow cytometry measurement value of the cell and the averaged first flow cytometry measurement value of the second calibrators is formed, and

- wherein, for formation of a normalized first flow cytometry measurement value for the second calibrator type, the ratio of the difference between the averaged first flow cytometry measurement value of the first calibrators of the second calibrator type and the averaged first flow cytometry measurement value of the second calibrators to the difference between the first flow cytometry measurement value of the cell and the averaged first flow cytometry measurement value of the second calibrators is formed.

3. Flow cytometry measurement method according to Claim 1 or 2, **characterized in that** there is provided for at least one first calibrator a first calibration factor which, with regard to the measurement signal for the first parameter, corresponds to the ratio between the measurement signal intensity of the first calibrator and the measurement signal intensity of a first reference calibrator having the target structure, **in that** a first measurement signal is measured for the first physical parameter of the first calibrator and the first flow cytometry measurement value of the first calibrator is formed from the first measurement signal and the first calibration factor, **in that** there is provided for the second calibrator a second calibration factor which, with regard to the measurement signal for the first parameter, corresponds to the ratio between the measurement signal intensity of the second calibrator and the measurement signal intensity of a second reference calibrator not having the target structure, and **in that** a second measurement signal is measured for the first physical parameter of the second calibrator and the first flow cytometry measurement value of the second calibrator is formed from the second measurement signal and the second calibration factor.

4. Flow cytometry measurement method according to any of Claims 1 to 3, **characterized in that** the fluid contains at least two different populations of cells which differ from one another such that the first flow cytometry measurement values of the cells of a first population lie within a first signal intensity range and the first flow cytometry measurement values of the cells of a second population lie within a second signal intensity range outside the first signal intensity range, and **in that** the signal intensity of the first flow cytometry measurement values of the first calibrator type is chosen such that it lies between the first and second signal intensity range.

5. Flow cytometry measurement method according to any of Claims 1 to 4, **characterized in that** the greatest dimension of the solid particles is smaller than 20 $\mu$m, more particularly smaller than 15 $\mu$m and preferably smaller than 10 $\mu$m and/or **in that** the smallest dimension of the solid particles is greater than 4 $\mu$m, more particularly greater than 5 $\mu$m and preferably greater than 6 $\mu$m.

6. Flow cytometry measurement method according to any of Claims 1 to 5, **characterized in that** the material of the solid particles is polystyrene, melamine, latex or a silicate.

7. Flow cytometry measurement method according to any of Claims 1 to 6, **characterized in that** the at least one second flow cytometry measurement value comprises a forward scattering measurement value for forward scattering of the energy beam that occurs in the measurement region and/or a sideward scattering measurement value for sideward scattering of the energy beam that occurs in the measurement region.

8. Flow cytometry measurement method according to any of Claims 1 to 7, **characterized in that**, both for the cells and for the first and second calibrators, respective measurements are made of at least one first flow cytometry measurement value for multiple measurement channels, **in that** at least a number of different labelling molecules that corresponds to the number of measurement channels is provided and is contacted with the analysis medium, and **in that** the at least one first calibrator has at least a number of target structures that corresponds to the number of measurement channels, which target structures respectively specifically bind to one of the different labelling molecules, if they come into contact with the labelling molecule in question.

9. Flow cytometry measurement method according to any of Claims 1 to 8, **characterized in that** the target structure on the first calibrators has at least one antigen which preferably comprises at least one protein and/or at least one peptide and/or at least one receptor and/or at least one allergen and/or at least one glycosylated protein and/or at least one liposaccharide and/or at least one oligosaccharide and/or at least one polysaccharide and/or at least one nucleic acid and/or at least one fragment and/or derivative of the aforementioned substances.

10. Flow cytometry measurement method according to any of Claims 1 to 9, **characterized in that** the target structure of the at least one first calibrator has been immobilized on the solid particle of the first calibrator via at least one activated carboxy group and/or at least one activated NH2 group.

**Revendications**

1. Procédé de mesure par cytométrie en flux, dans lequel un milieu d'analyse est fourni qui comporte un fluide et des cellules biologiques à classer contenues à l'intérieur de celui-ci,

   - au moins une molécule de marquage étant fournie et mise en contact avec le milieu d'analyse de manière que la molécule de marquage puisse se lier spécifiquement à au moins une structure cible, située à la surface des cellules, si la cellule comporte cette structure cible,
   - un écoulement fluide du milieu d'analyse étant généré dans lequel les cellules atteignent individuellement une zone de mesure d'un faisceau d'énergie et/ou d'un champ électrique,
   - une première valeur de mesure par cytométrie en flux d'au moins un premier paramètre physique et une deuxième valeur de mesure par cytométrie en flux d'un deuxième paramètre physique étant acquises pour les cellules individuelles situées dans la zone de mesure,
   - au moins le premier paramètre étant un rayonnement par fluorescence que l'au moins une molécule de marquage émet lors de l'excitation avec le faisceau d'énergie ou le champ électrique,
   - les cellules étant classées sur la base des valeurs de mesure par cytométrie en flux,
   - au moins deux types d'étalonneur étant fournis par des premiers étalonneurs et au moins un deuxième étalonneur,
   - les premiers étalonneurs et l'au moins un deuxième étalonneur comportant chacun une particule solide faite d'une matière minérale et/ou polymère insoluble dans l'eau et les particules solides des premiers étalonneurs et de l'au moins un deuxième étalonneur concordant en termes de forme, de taille et de matière,
   - les premiers étalonneurs comportant sur leur surface au moins une structure cible qui concorde avec l'au moins une structure cible des cellules et qui est immobilisée sur les particules solides des premiers étalonneurs,
   - l'au moins un deuxième étalonneur ne comportant pas cette structure cible,
   - les premiers étalonneurs et l'au moins un deuxième étalonneur étant mélangés au milieu d'analyse avant l'acquisition des valeurs de mesure par cytométrie en flux de manière qu'au moins une molécule de marquage, située dans le milieu d'analyse, se lie à l'au moins une structure cible des premiers étalonneurs,
   - les étalonneurs dans le flux de fluide étant amenés individuellement l'un après l'autre dans la zone de mesure,
   - des premières et deuxièmes valeurs de mesure par cytométrie en flux correspondantes étant acquises pour les premiers étalonneurs et l'au moins un deuxième étalonneur comme pour les cellules,
   - le paramètre associé à la deuxième valeur de mesure par cytométrie en flux des cellules et à la deuxième valeur de mesure par cytométrie en flux des étalonneurs étant sélectionné de manière que les étalonneurs puissent être distingués des cellules sur la base des deuxièmes valeurs de mesure par cytométrie en flux,
   - les étalonneurs des différents types d'étalonneur différant les uns des autres, en particulier en ce qui concerne la densité de couverture de surface et/ou la disposition de leur au moins une structure cible sur la surface de la particule solide de manière que les premières valeurs de mesure par cytométrie en flux d'un premier type d'étalonneur aient une intensité de signal supérieure à l'intensité de signal des premières valeurs de mesure par cytométrie en flux des deuxièmes étalonneurs et inférieure à l'intensité de signal des premières valeurs de mesure par cytométrie en flux d'un deuxième type d'étalonneur,
   - pour former une première valeur de mesure par cytométrie en flux normalisée pour le premier type d'étalonneur, la différence entre la première valeur de mesure par cytométrie en flux du premier étalonneur du premier type d'étalonneur et la première valeur de mesure par cytométrie en flux du deuxième étalonneur étant mise en rapport avec la différence entre la première valeur de mesure par cytométrie en flux de la cellule et la première valeur de mesure par cytométrie en flux du deuxième étalonneur et
   - pour former une première valeur de mesure par cytométrie en flux normalisée pour le deuxième type d'étalonneur, la différence entre la première valeur de mesure par cytométrie en flux du premier étalonneur du deuxième type d'étalonneur et la première valeur de mesure par cytométrie en flux du deuxième étalonneur étant mise en rapport avec la différence entre la première valeur de mesure par cytométrie en flux de la cellule et la première valeur de mesure par cytométrie en flux du deuxième étalonneur.

**2.** Procédé de mesure par cytométrie en flux, dans lequel un milieu d'analyse est fourni qui comporte un fluide et des cellules biologiques à classer contenues à l'intérieur de celui-ci,

- au moins une molécule de marquage étant fournie et mise en contact avec le milieu d'analyse de manière que la molécule de marquage puisse se lier spécifiquement à au moins une structure cible, située à la surface des cellules, si la cellule comporte cette structure cible,
- un écoulement fluide du milieu d'analyse étant généré dans lequel les cellules atteignent individuellement une zone de mesure d'un faisceau d'énergie et/ou d'un champ électrique,
- une première valeur de mesure par cytométrie en flux d'au moins un premier paramètre physique et une deuxième valeur de mesure par cytométrie en flux d'un deuxième paramètre physique étant acquises pour les cellules individuelles situées dans la zone de mesure,
- au moins le premier paramètre étant un rayonnement par fluorescence que l'au moins une molécule de marquage émet lors de l'excitation avec le faisceau d'énergie ou le champ électrique,
- les cellules étant classées sur la base des valeurs de mesure par cytométrie en flux,
- au moins deux types d'étalonneur étant fournis qui comportent chacun une pluralité de premiers étalonneurs identiques et une pluralité de deuxièmes étalonneurs identiques,
- les premiers étalonneurs et les deuxièmes étalonneurs comportant chacun une particule solide faite d'une matière minérale et/ou polymère insoluble dans l'eau et les particules solides des premiers et deuxièmes étalonneurs concordant en termes de forme, de taille et de matière,
- les premiers étalonneurs comportant sur leur surface au moins une structure cible qui concorde avec l'au moins une structure cible des cellules et qui est immobilisée sur les particules solides des premiers étalonneurs,
- les deuxièmes étalonneurs ne comportant pas cette structure cible,
- les premiers étalonneurs et deuxièmes étalonneurs étant mélangés au milieu d'analyse avant l'acquisition des valeurs de mesure par cytométrie en flux de manière qu'au moins une molécule de marquage, située dans le milieu d'analyse, se lie à l'au moins une structure cible des premiers étalonneurs,
- les étalonneurs dans le flux de fluide étant amenés individuellement l'un après l'autre dans la zone de mesure,
- des premières et deuxièmes valeurs de mesure par cytométrie en flux correspondantes étant acquises pour les premiers et deuxièmes étalonneurs comme pour les cellules,
- une première valeur de mesure par cytométrie en flux moyenne pour les premiers étalonneurs du type d'étalonneur concerné étant formée à partir des premières valeurs de mesure par cytométrie en flux des premiers étalonneurs de chaque type d'étalonneur et une première valeur de mesure par cytométrie en flux moyenne pour les deuxièmes étalonneurs étant formée à partir des premières valeurs de mesure par cytométrie en flux des deuxièmes étalonneurs,
- le paramètre associé à la deuxième valeur de mesure par cytométrie en flux des cellules et à la deuxième valeur de mesure par cytométrie en flux des étalonneurs étant sélectionné de manière que les étalonneurs puissent être distingués des cellules sur la base des deuxièmes valeurs de mesure par cytométrie en flux,
- les étalonneurs des différents types d'étalonneur différant les uns des autres, en particulier en ce qui concerne la densité de couverture de surface et/ou la disposition de leur au moins une structure cible sur la surface de la particule solide de manière que les premières valeurs de mesure par cytométrie en flux d'un premier type d'étalonneur aient une intensité de signal supérieure à l'intensité de signal des premières valeurs de mesure par cytométrie en flux des deuxièmes étalonneurs et inférieure à l'intensité de signal des premières valeurs de mesure par cytométrie en flux d'un deuxième type d'étalonneur,
- pour former une première valeur de mesure par cytométrie en flux normalisée pour le premier type d'étalonneur, la différence entre la première valeur de mesure par cytométrie en flux moyenne du premier étalonneur du premier type d'étalonneur et la première valeur de mesure par cytométrie en flux moyenne du deuxième étalonneur étant mise en rapport avec la différence entre la première valeur de mesure par cytométrie en flux de la cellule et la première valeur de mesure par cytométrie en flux moyenne du deuxième étalonneur et
- pour former une première valeur de mesure par cytométrie en flux normalisée pour le deuxième type d'étalonneur, la différence entre la première valeur de mesure par cytométrie en flux moyenne du premier étalonneur du deuxième type d'étalonneur et la première valeur de mesure par cytométrie en flux moyenne du deuxième étalonneur étant mise en rapport avec la différence entre la première valeur de mesure par cytométrie en flux de la cellule et la première valeur de mesure par cytométrie en flux moyenne du deuxième étalonneur.

**3.** Procédé de mesure par cytométrie en flux selon la revendication 1 ou 2, **caractérisé en ce qu'**il est prévu pour au moins un premier étalonneur un premier facteur d'étalonnage qui correspond, en ce qui concerne le signal de mesure du premier paramètre, au rapport entre l'intensité de signal de mesure du premier étalonneur et l'intensité de signal de mesure d'un premier étalonneur de référence comportant la structure cible, **en ce qu'**un premier signal de mesure est acquis pour le premier paramètre physique du premier étalonneur et la première valeur de mesure par cytométrie en flux du premier étalonneur est formée à partir du premier signal de mesure et du premier facteur d'étalonnage, **en ce qu'**il est prévu pour le deuxième étalonneur un deuxième facteur d'étalonnage qui correspond, en ce qui concerne le signal de mesure du premier paramètre, au rapport entre l'intensité de signal de mesure du deuxième étalonneur et l'intensité de signal de mesure d'un deuxième étalonneur de référence qui ne comporte pas la structure cible, et **en ce qu'**un deuxième signal de mesure est acquis pour le premier paramètre physique du deuxième étalonneur et la première valeur de mesure par cytométrie en flux du deuxième étalonneur est formé à partir du deuxième signal de mesure et du deuxième facteur d'étalonnage.

4. Procédé de mesure par cytométrie en flux selon l'une des revendications 1 à 3, **caractérisé en ce que** le fluide contient au moins deux populations différentes de cellules qui diffèrent l'une de l'autre de manière que les premières valeur de mesure par cytométrie en flux des cellules d'une première population soient situées dans une première zone d'intensité de signal et les premières valeur de mesure par cytométrie en flux des cellules d'une deuxième population soient situées dans une deuxième zone d'intensité de signal à l'extérieur de la première zone d'intensité de signal, et **en ce que** l'intensité de signal des premières valeurs de mesure par cytométrie en flux du premier type d'étalonneur est sélectionnée de manière à être située entre les première et deuxième zones d'intensité de signal.

5. Procédé de mesure par cytométrie en flux selon l'une des revendications 1 à 4, **caractérisé en ce que** la plus grande dimension des particules solides est inférieure à 20 $\mu$m, notamment inférieure à 15 $\mu$m et de préférence inférieure à 10 $\mu$m et/ou **en ce que** la plus petite dimension des particules solides est supérieure à 4 $\mu$m, en particulier supérieure à 5 $\mu$m et de préférence supérieure à 6 $\mu$m.

6. Procédé de mesure par cytométrie en flux selon l'une des revendications 1 à 5, **caractérisé en ce que** la matière des particules solides est le polystyrène, la mélamine, le latex ou un silicate.

7. Procédé de mesure par cytométrie en flux selon l'une des revendications 1 à 6, **caractérisé en ce que** l'au moins une deuxième valeur de mesure par cytométrie en flux est une valeur de mesure de diffusion vers l'avant pour une diffusion vers l'avant du faisceau d'énergie se produisant dans la zone de mesure et/ou une valeur de mesure de diffusion latérale pour une diffusion latérale du faisceau d'énergie se produisant dans la zone de mesure.

8. Procédé de mesure par cytométrie en flux selon l'une des revendications 1 à 7, **caractérisé en ce qu'**au moins une première valeur de mesure par cytométrie en flux est acquise aussi bien pour les cellules que pour les premiers et deuxièmes étalonneurs pour une pluralité de canaux de mesure, **en ce qu'**au moins un certain nombre de molécules de marquage différentes, correspondant au nombre de canaux de mesure, est prévu et mis en contact avec le milieu d'analyse, et **en ce que** l'au moins un premier étalonneur comporte au moins un certain nombre de structures cibles, correspondant au nombre de canaux de mesure, qui se lient chacune spécifiquement à l'une des différentes molécules de marquage lorsqu'elles sont en contact avec la molécule de marquage concernée.

9. Procédé de mesure par cytométrie en flux selon l'une des revendications 1 à 8, **caractérisé en ce que** la structure cible sur les premiers étalonneurs comporte au moins un antigène qui comprend de préférence au moins une protéine et/ou au moins un peptide et/ou au moins un récepteur et/ou au moins un allergène et/ou au moins une protéine glycosylée et/ou au moins un liposaccharide et/ou au moins un oligosaccharide et/ou au moins un polysaccharide et/ou au moins un acide nucléique et/ou au moins un fragment et/ou un dérivé des substances susmentionnées.

10. Procédé de mesure par cytométrie en flux selon l'une des revendications 1 à 9, **caractérisé en ce que** la structure cible de l'au moins un premier étalonneur est immobilisée sur la particule solide du premier étalonneur par le biais d'au moins un groupement carboxyle activé et/ou d'au moins un groupement NH2 activé.

MFI 0,0 µg

MFI 1,0 µg / 2x10^6
Festkörperpartikel

MFI 2,0 µg / 2x10^6
Festkörperpartikel

n

n

n

800

600

400

200

0

800

600

400

200

0

800

600

400

200

0

$10^1$  $10^2$  $10^3$  $10^4$

$10^1$  $10^2$  $10^3$  $10^4$

$10^1$  $10^2$  $10^3$  $10^4$

PE

PE

PE

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2010015643 A1

**In der Beschreibung aufgeführte Nicht-Patentliteratur**

- **HERMANSON, GREG T.** Bioconjugate Techniques. 2013